(19) Europäisches Patentamt
European Patent Office
Office européen des brevets

(11) **EP 3 851 129 A1**

(12) **EUROPEAN PATENT APPLICATION**

(43) Date of publication:
21.07.2021 Bulletin 2021/29

(51) Int Cl.:
*A61L 27/16* (2006.01)    *A61L 27/56* (2006.01)

(21) Application number: 20152766.0

(22) Date of filing: 20.01.2020

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
KH MA MD TN

(71) Applicant: National University of Ireland Galway
Galway (IE)

(72) Inventors:
• YAGUE, Marc Antoni Fernadez
H91 TK33 Galway (ES)
• Biggs, Manus Jonathan Paul
Galway, H91 TK33 (IE)
• Pandit, Abhay
Galway, H91 TK33 (IE)
• Sunny, Abbah
Galway, H91 TK33 (IE)

(74) Representative: Murgitroyd & Company
Murgitroyd House
165-169 Scotland Street
Glasgow G5 8PL (GB)

(54) **TENDON OR LIGAMENT SCAFFOLDS FORMED FROM ELECTRO-SPUN PTFE AND METHOD FOR OBTAINING THEM**

(57) The present application relates to a tendon or ligament repair implant comprising a biocompatible scaffold, wherein the scaffold is formed from electro-spun PTFE wherein the electro-spun PTFE comprises a plurality of aligned nanoscale PTFE fibres and a method for making the same. Suitably the method may comprise the steps

a) mixing a dispersion of PTFE particles in water with a fibre forming agent,
b) electrospinning the mixture of a) onto a collector; and
c) sintering the electrospun material to form a biocompatible scaffold.

Figure 1.

**Description**

Field of the Invention

The present invention is directed to an implantable scaffold for use in tendon or ligament repair, wherein the scaffold is formed of electrospun PTFE.

Background of Invention

Sprains, tears or ruptures of ligaments and tendons of the knee, elbow, hand, shoulder, wrist and jaw are relatively common injuries. The Achilles or the rotator cuff tendons are of particular concern as these often have poor healing capabilities.

In particular, complete tears of the Achilles or Rotator Cuff tendons are one of the leading causes of activity-related disability and one of the most significant unmet clinical challenges (with 20 to 90% repair failure rates just 3 months post-surgery). Achilles tendon is the largest tendon in the body and connects the muscles of the calf (gastrocnemius and soleus) to bone (calcaneus). During its function, the Achilles tendon is able to withstand up to eight times body weight and store up to 40% of deformation energy during gait. This extreme mechanical demand is borne by a highly organized extracellular matrix that is composed of resilient fibrous cords of collagen type I. However, due to the low content of cellular components and lack of vascular networks, the regenerative capacity of tendon is very poor and generally after an injury, repaired tendons never match the biomechanical properties of native tendon tissue.

Normal tendon healing after an injury results in the formation of poor scar tissue followed by gradual remodeling over time. Briefly, the natural healing of tendon involves three overlapping phases: inflammatory phase that occurs immediately after injury; a proliferate phase starts within the first week and results in the formation of a scar tissue that bridges the two ends of the ruptured tendon; and finally the remodeling phase begins around 4 to 6 weeks after injury. During the remodeling phase, there is a gradual increase of collagens organization, cross-linking level and mechanical properties followed by a decrease in matrix production and cellular infiltration and activity. In addition to that, expression of phenotypic markers of tendon fibroblasts, such as tenomodulin or scleraxis also decreases.

Autografts, allografts, xenografts and degradable and non-degradable synthetic prostheses have been used in tendon and ligament repair. Problems associated with the use of existing synthetic prosthesis have led towards the development of functional and biocompatible equivalents.

Complete tendon repair can go on up to two years and one the great interests in the regenerative medicine field is to develop new treatments that can accelerate the repair process. Current treatments involve (1) direct end-to-end repair when possible, (2) tendon grafts (GraftJacket®, TissueMend®, Restore™, CuffPatch®, Permacol™, OrthADAPT®, Bio-Blanket® or (3) synthetic polymers (Gore-Tex® patch WL, Lars® ligament, Leeds-Keio® or Poly-tape®, and Artelon®, & Sportmesh™). Repaired tendons using synthetic scaffolds from materials such as Teflon (PTFE) present the best clinical outcomes due to their permanent mechanical support. However, they are also associated with limited long-term tissue compatability and poor tissue integration. Common clinical complications include inflammatory processes associated with foreign body reaction and adhesion formations, preventing their clinical adoption. In summary, current devices using synthetic materials focus primarily on early restoration of function but fail to address the underlying mechanisms of long term related issues as adhesion formation or poor mechanical properties of healed tissues due to loss of ECM organization.

Tendon is a unique tissue that connects muscle to bone and therefore is under continuous mechanical loading. As noted, this mechanical stress is borne by a highly organised extracellular matrix composed of collagen type I. Moreover, collagen has been determined to be a piezoelectric material.

Attempts to control ECM organization and maintain tendon function have involved the use of fibrous scaffolds, typically scaffolds containing aligned nanoscale fibers to recreate the native structured ECM of tendon. Moreoever, electrical stimulation has been used to mimic the naturally occurring electrical current to facilitate wound healing.

However, there still remains a need for a scaffold to generate more fully functional bioengineered ligaments and tendons.

**Summary of the Invention**

The inventors considered mechanical, electrical and topographical features on tendon cell function and developed a dynamic bioreactor system to consider electromechanical cues on tenocytes morphology and function in 3D models. Based on this work, a collagen piezoelectric analogue was developed using PVDF-TrFE which undergoes electrical polarisation in response to mechanical loading. There is provided a new biomimetic scaffold comprised of aligned nanoscale fibres made of PVDF-TrFE. The fabrication method chosen was electrospinning due to its ability to mimic the fibrous structure of the native tendon. Furthermore, by altering the collector rotational speed, it was determined that fibre organisation could be controlled.

Accordingly, a first aspect of the present invention provides a tendon or ligament repair implant comprising a biocompatible scaffold, wherein the scaffold is formed from electro-spun PTFE wherein the electro-spun PTFE comprises a plurality of aligned nanoscale PTFE fibres.

Suitably, the implant comprises an alignment of the fibres of at least 90%. Suitably, the fibers may be provided by cold drawing to provide a total alignment of at least 90%, decreased fiber diameter and that practically all fibers carry load during force transmission to provide mechanical strength to the implant.

Suitably, the biocompatible scaffold may have a porous structure configured to allow cell and tissue ingrowth. Suitably the PTFE of the implant may have a porosity between 60 to 80%.

Suitably, the scaffold may have a percentage elongation of at least 90% and a tensile strength of between 1.35 to 8 MPa. Suitably, the scaffold may have a lamina or tubular shape or complex shape. Suitably the outer surface of the scaffold may comprise hydrophilic groups, optionally wherein the hydrophilic groups are acrylic acid.

Suitably the scaffold may further comprise a nanometric polyacrylic acid outer layer. Suitably the implant may further comprise nanoparticles, for example wherein the nanoparticles provide enhanced mechanical strength and / or alter the elastic modulus of the implant. In embodiments wherein the implant comprises nanoparticles, the nanoparticles may comprise boron nitride nanotubes or carbon nanotubes with length range between 10 nm and 10 $\mu$m.

According to a second aspect of the present invention there is provided a method of preparing a tendon or ligament repair implant, the method comprising:

a) mixing a dispersion of PTFE particles in water with a fibre forming agent, and electrospinning the mixture onto a collector; and

b) sintering the electrospun material to form a biocompatible scaffold.

As in the present method the fibers are dried in an oven for sintering, the void volume between the PTFE particles, and for example the PEO between the PTFE particles will disapper which would decrease the size of the scaffold. The decrease may be about a 30 to 40% reduction. To accommodate this change without resulting in fibre ruptures, a stretchable holder for the scaffold or release of the scaffold from the collector so it can slide while it is changing the volume is required. Thus, the scaffold is not left in a normal collector for electrospinning and a stretchable holder may be used.

Suitably the fibre forming agent may be poly(ethylene oxide) (PEO) or PVA, optionally having an average molecular weight of between 1 to 1,000,000 kDa, optionally about 300KDa.

Suitably wherein the fibre forming agent is poly(ethylene oxide) (PEO), the PTFE:PEO ratio may be between 0.01 and 0.05, and optionally the mixture has a maximum viscosity of 1 Pa.s (10 poise).

Suitably, the mixture may be electrospun in a relative humidity level of no greater than 60%, optionally between 50 to 60%.

Suitably in embodiments electrospinning the material can comprise discharging the mixture from a needle of a syringe at a flow rate of no more than 1.25 mL/hr, optionally 1.0 mL/hr. Suitably in embodiments of the electro-spraying method, the distance between the needle and the collector can be 10 to 15 cm, optionally 10 to 12 cm.

Suitably the electrospun material can be sintered at a temperature of 385°C.

Suitably wherein sintering is provided, the step of sintering the electrospun material may comprise:

a) heating at 10 °C/min to 350°C and holding for at least 5 minutes; and

b) heating at 10 °C/min to 385°C and holding for at least 5 minutes.

Suitably the method may comprise a drying step prior to sintering, wherein when there is provided a step of drying the electrospun material prior to sintering, the drying may be provided for 2 to 24 hours at 70°C.

Suitably the method may further comprise the step of functionalising an outer surface of the scaffold with hydrophilic groups. In embodiments the hydrophilic groups may be acrylic acid. Suitably functionalising the outer surface of the scaffold may comprise:

treating the surface with oxygen plasma;

immersing the plasma treated samples in methanol to wet the surface; and

heating the samples under reflux in an aqueous solution of acrylic acid.

In embodiments of the method, the treating step can be with oxygen plasma for 30s to 30 minutes. In embodiments, the step of heating under reflux can be in an aqueous solution of acrylic acid is at 90°C for 1 to 30 min using acrylic acid at 20%.

In the electrospray method, the collector can have a length of between 1 cm to 10 cm. In embodiments, the collector can be a rotatable mandrel, optionally wherein the temperature is between 30 to 90°C and optionally wherein the mandrel is rotating at up to 4000 rpm, suitably about 2000 rpm.

Suitably, the method may further comprise incorporating boron nitride nanotubes or carbon nanotubes with length range between 10 nm and 10 μm.

In embodiments of the method, the method can include a step of cold drawing.

The post-treatment cold drawing step is provided to obtain highly aligned fibres. At the velocities utilised, the collector does not provide high fiber alignment. Due to the extremely low mechanical integrity of the fibres, the rpms are limited as they produce centripetal forces that cause fiber rupture. Beyond 2000rpm, the fibers can start to break and cause the scaffold to break apart. The inventors have determined a velocity of greater than 3600 rpm for the collector used of 10 cm in length provides less than 90% alignment of fibres. The cold drawing step allows the increase in the alignment of fibres.

The cold drawing step may be carried out to align further the fibers and increase mechanical strength of the scaffold and may comprises the steps of

- extruding the electrospun PTFE having a predetermined thickness and length, a proximal end and a distal end, the proximal end having a temperature;
- drawing the distal end to a predetermined length while maintaining the temperature of the scaffold below the glass transition temperature of PTFE during the drawing, the wall thickness of the proximal and distal ends form a waist

Suitably, in the drawing step of the method, the scaffold can be maintained at a temperature of no more than approximately room temperature. In embodiments, during the drawing step, the scaffold can be stretched to 12% suitably, the stretch rate may be between 5% to 50% per second.

In embodiments, the stretch rate may be approximately 5% per second and the fibers may be aligned between 60 to 90% parallel to the direction of stretching, optionally wherein the fibers get aligned at 90% parallel to the direction of stretching.

Brief Description of Figures

Embodiments of the present invention will now be provided by way of example only with reference to the figures below, in which:

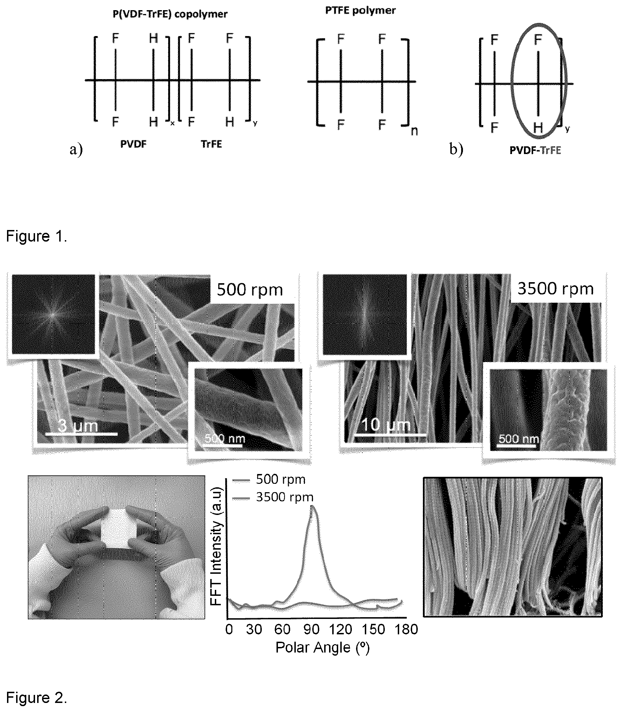

Figure 1 illustrates a) Schematic of the molecular structure of P(VDF-TrFE) copolymer and PTFE polymer. Note the structure of PTFE with four polar covalent Carbon-Fluorine bonds making it extremely stable and therefore Non-Piezoelectric. b) Within the P(VDF-TrFE) copolymer, the -TrFE component exhibits a strong dipole across the Hydrogen to Fluorine bond (circled).

Figure 2 illustrates morphology of the scaffolds obtained by electrospinning collected at low (500 rpm) and high (3500 rpm) rotational speeds. Fast Fourier Transform (FFT) spectra show a broad distribution of intensities for low speed (500 rpm) scaffolds. In contrast, FFT spectra for scaffolds fabricated at high rotational speeds (3500 rpm) shows a clear peak demonstrating the highly repetitive and organised structure. Taking as template the native tendon tissue, it was observed that at high rpm fibre alignment was significantly increased as shown by FFT intensity.

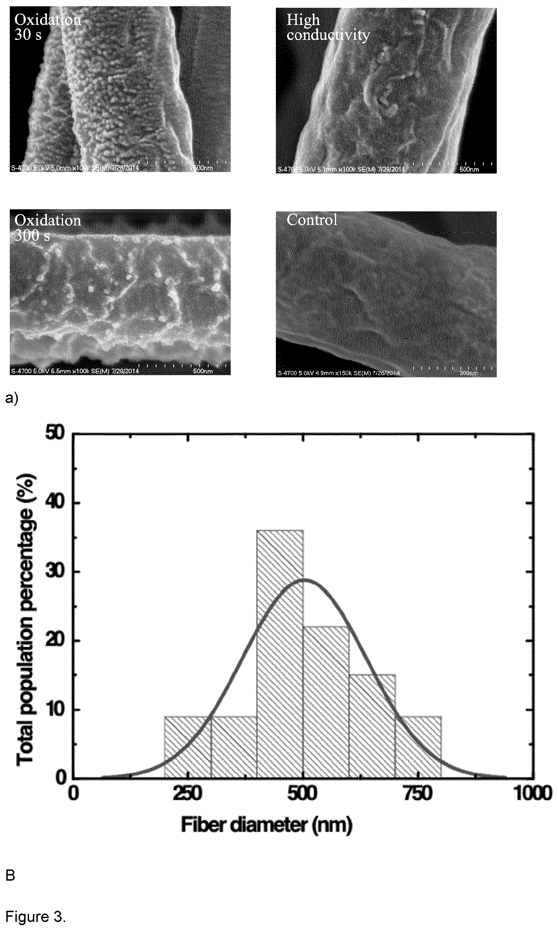

Figure 3 illustrates SEM images showing a different range of surface morphologies. a) Effect on fibre surface morphology of plasma treatment for 30 or 300 seconds and the effect of increasing conductivity on surface morphology b) Fibre diameter distribution.

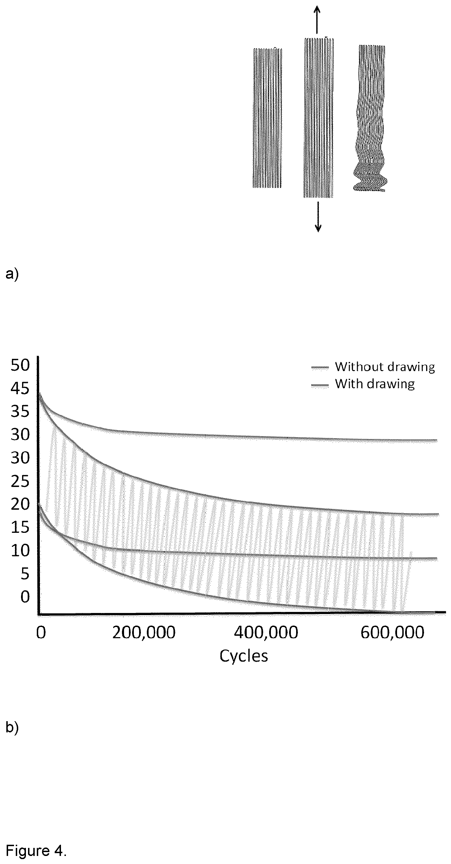

Figure 4 illustrates the effect of cold drawing in stress relation. a) Schematic of the effect of cyclic loading of electrospun scaffolds. As a result of loading the entangled fibres tend to align increasing the overall length of the scaffold and reducing the experienced stress over the time b) Stress measurement of scaffolds after 600,000 loading cycles (corresponding to the equivalent of applying strain cycles 8 hours/day for 20 days at 1 Hz). Stress relaxation is significantly reduced after cold drawing.

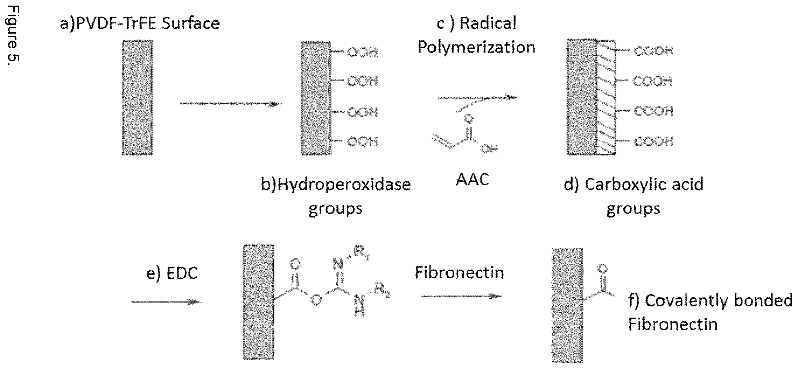

Figure 5 illustrates the functionalizing process illustrated via a stepwise mechanism. a) First, the square sample holder was placed in an RF (radio frequency) plasma chamber and treated with oxygen plasma for 45 seconds. b) Hydroperoxides produce secondary radicals that initiate the c) polymerisation of Acrylic acid (AAc) process. d) PAAc acts as a spacer on the PVDF surface and offers carboxylic acid groups. e) Carbodiimide compound EDC/NHS was used for crosslinking between primary amine from fibronectin and carboxylic acids. f) Fibronectin was then covalently bound to the PVDF-pAAc surface using N-(3-Dimethylaminopropyl)-N'-ethylcarbodiimide hydrochloride (EDC).

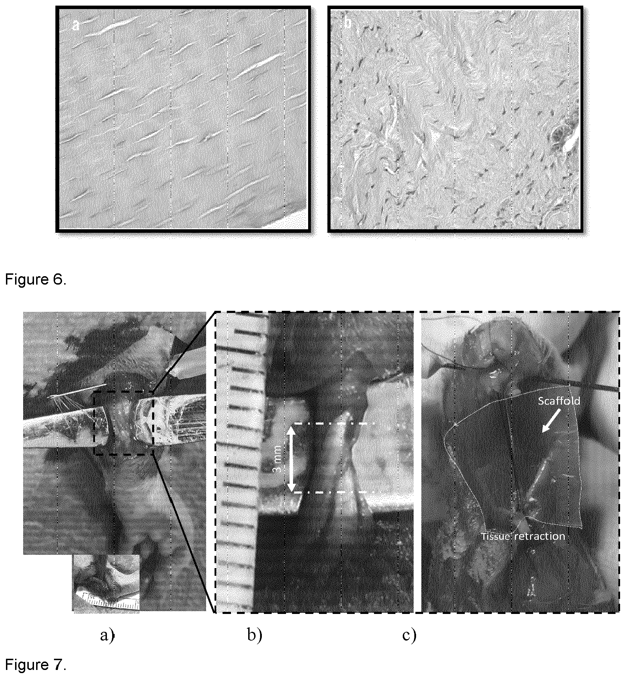

Figure 6 illustrates H&E pictures of Histological differences between intact and healed tendons after an injury - Tendon structure is lost after healing and is associated to hyper cellularity and poor mechanical performance due to immature collagen III overexpression using s piezoelectric scaffold made from electro-spun PVDF-TrFE. Using structural features that more closely mimic the natural tendon, these scaffolds provide enhanced resilience and thus improve the biomechanical performance. Furthermore, the piezoelectric scaffolds unique electrical response to physiological mechanical loading is considered to aid the repair of the tendon tissue through the activation of definite regeneration pathways. To apply mechanical forces to the tendons and piezoscaffolds to activate their electrical response *in vivo* an adapted protocol of treadmill running was used.

Figure 7 illustrates Construct implantation, a) Tendon is exposed b) tendon is measured and 3 mm is removed c) after tissue retraction a 6 mm gap is generated and the implant is attached to both free ends using a modified Kessler technique.

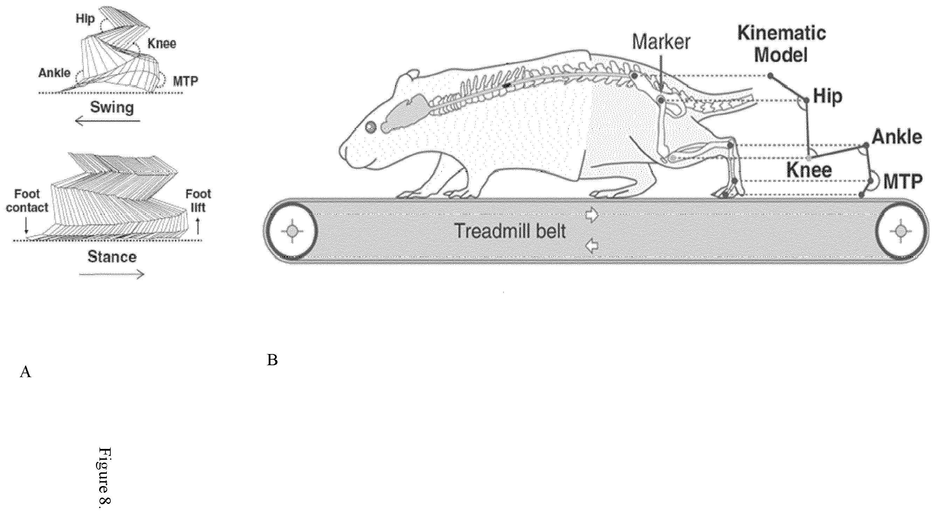

Figure 8 illustrates a) Schematic representation of the kinematic model extracted from the ilium, hip, ankle, knee, MTP and 3$^{rd}$ toe markers on bare skin. b) Schematic representation of the sagittal view of the walkway corresponding to the horizontal plane recorded during experiment.

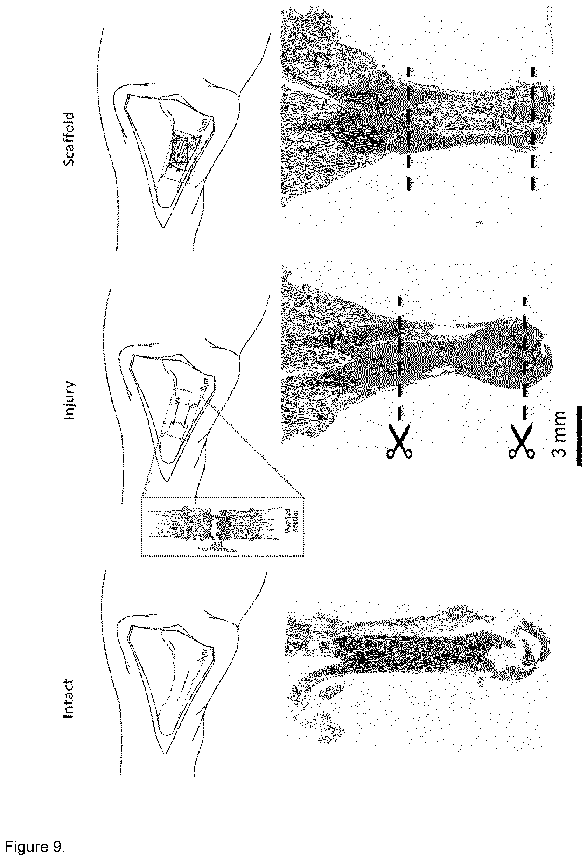

Figure 9 illustrates schematic representation and histological pictures of samples stained with Herovici's of a) intact tendon b) injured tendon repaired with sutures and c) injured tendon repaired with biomaterial. The images showed a complete closure of the tendon defect area after 2 weeks post injury

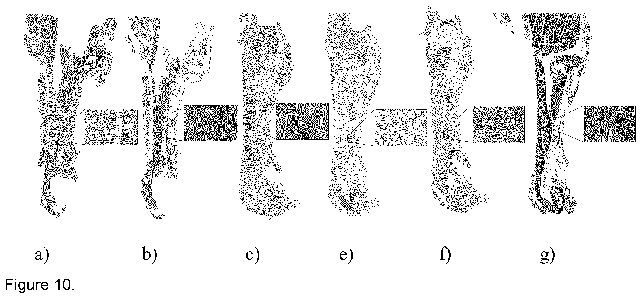

Figure 10 illustrates histological images of intact tendon a) H&E staining showing cell organization and fiber bundles. b) Herovici staining showing difference between collagen III and I. c) Trichrome Masson showing fibrous structure and collagen type I appears completely stained with fucsin (red) d) Alcian blue showing mucopolysaccharides surrounding cells e) Picrosirius red showing structured fiber organization f) Safranin O-stained sections showing absence of any ectopic ossification and cartilage formation in the tendon-bone insertion.

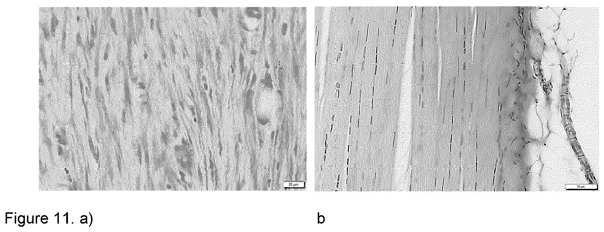

Figure 11 illustrates H&E images of a) injured tendon sample (1 week) and b) intact tendon sample showing differences in cell morphology. Pink, tissue matrix; purple, cytoplasm; blue, nuclei.

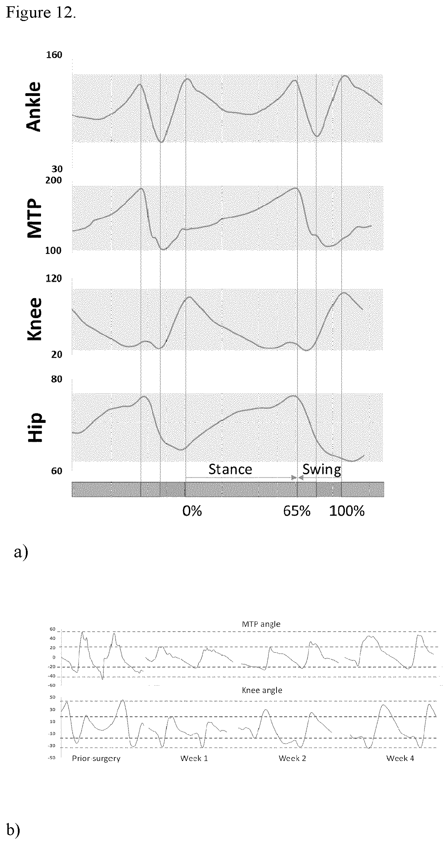

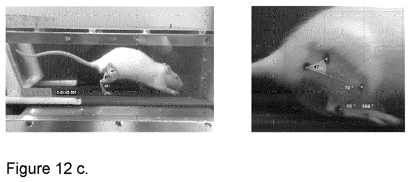

Figure 12 illustrates a) Gait analysis data of animal pre-surgery: A total of four different joint angles were measured (Ankle, MTP, Knee and Hip). b) Experimental MTP and Knee angles before and after surgery at weeks 1, 2 and 4. Different amplitudes were obtained for animals before and after surgery (injury) at all time points.

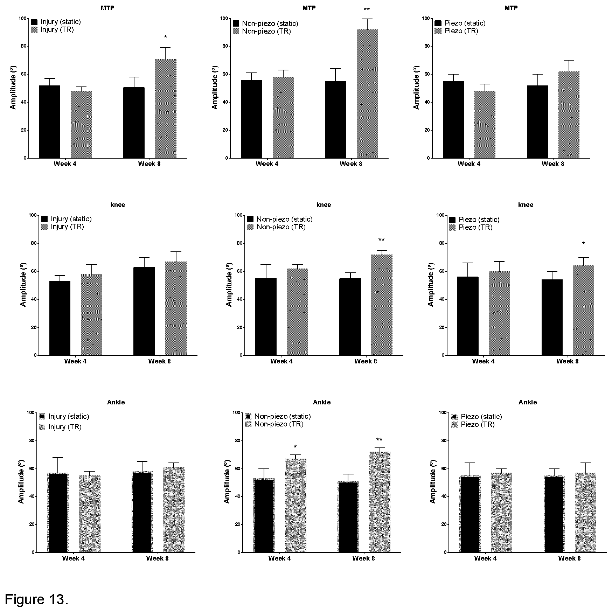

Figure 13 illustrates Mean and standard deviation of the amplitude from the injury, non-piezo and piezo groups (static and TR) at weeks 4 and 8 after injury (* p<005, ** p<0.01). It is observed that the group of non-piezo (TR) showed significant functional recovery in the ankle, MTP and knee joints after 4 and 8 weeks.

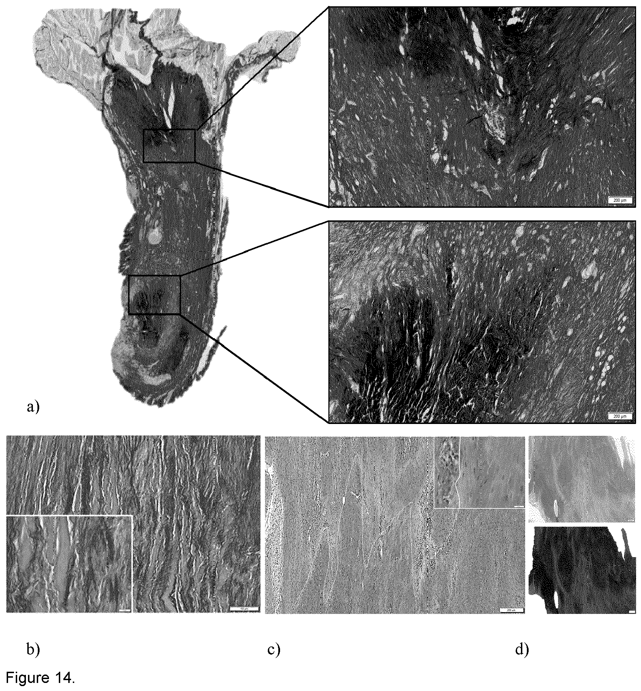

Figure 14 illustrates a) Picrosiruis red stained sample 1 week post tendon transection modified digitally to enhance the contrast between the different phases (granulation in red and native tendon tissue in purple). Macroscopic and 10x magnifications of areas showing the interphase between native tendon and newly formed granulation tissue. b) Herovici stained sample 2 weeks post tendon transection showing the persistent breakdown of the tendon fibrils at the cut ends c) Alcian blue stained sample 2 weeks post tendon transection showing large bundle of fibers of collagen separated due to disruption of organized tissue. d) Alcian blue stained samples 2 weeks after injury contrasted digitally using gray scale. The tendon end stump is clearly separated from the granulation tissue.

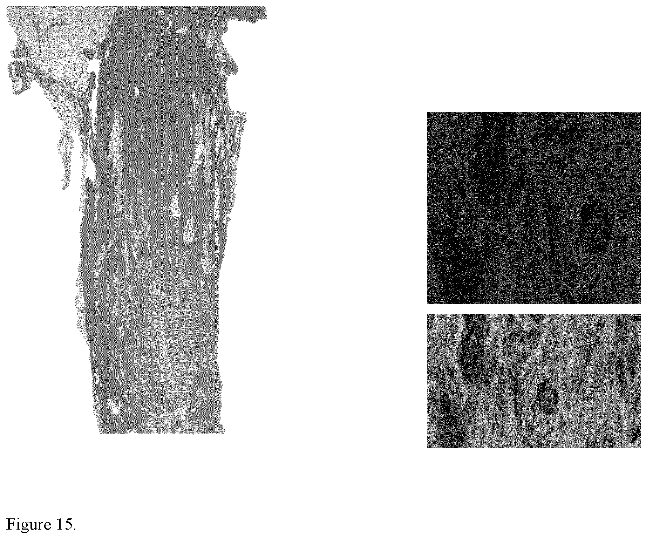

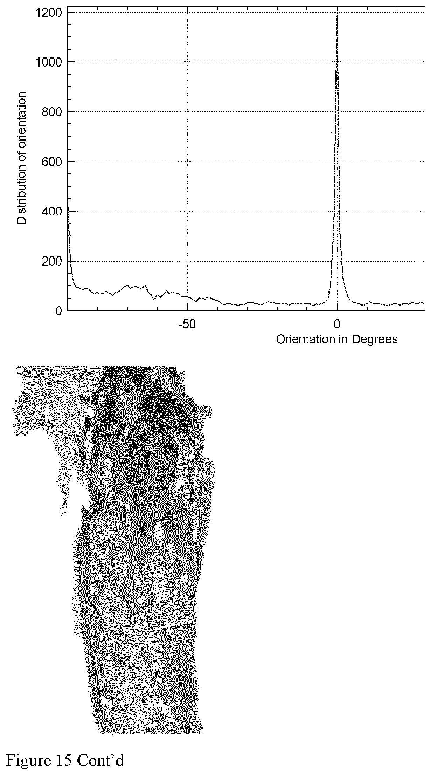

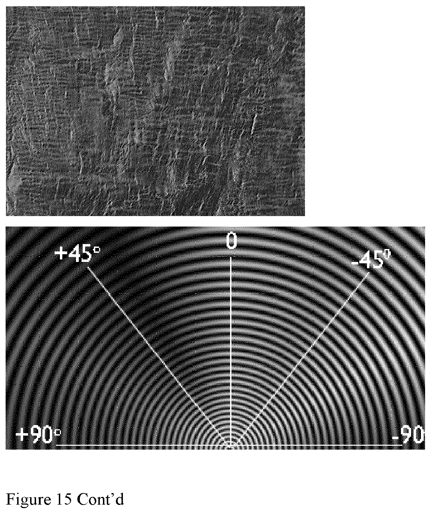

Figure 15 illustrates Picrosirius red stained sample with (right) and without polarization (left). Digital analysis shows that distribution of the orientation of fibres is broad and therefore disporganized. Circular colour map shows the correlation between orientation and colour code. The polarized image demonstrates that the deposited collagen is highly disorganized and shows poor birefringence in suture-aided tendon healed characteristic of collagen type III

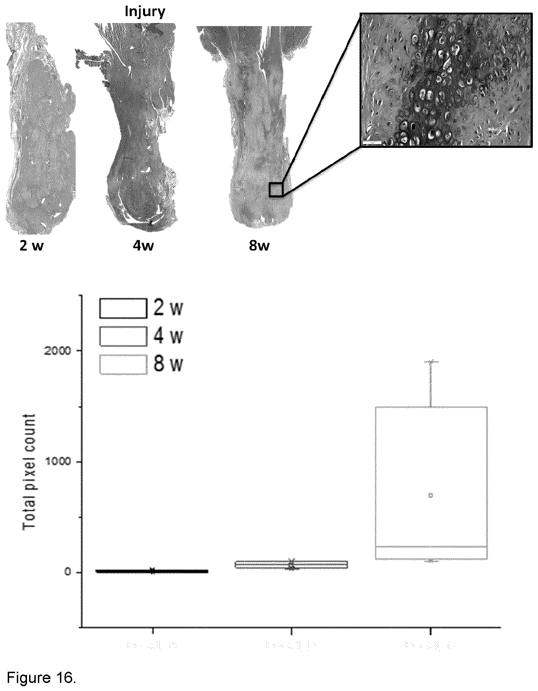

Figure 16 illustrates a) Safranin-O stained samples at 2, 4 and 8 weeks after injury without scaffold. After 8 weeks it is noticeable the calcified areas in the mid substance. b) Chondrification area for all samples after 2,4 and 8 weeks PTI. Histomorphometric measurements showed a significant increase on the amount of area undergoing chondrification after 8 weeks post-injury.

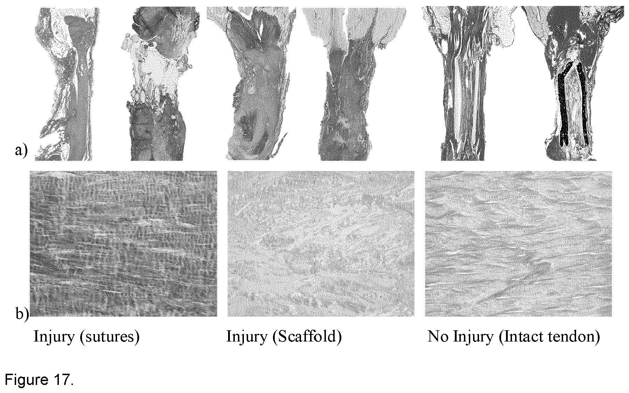

Figure 17 illustrates a) Picrosirius red staining of different samples namely from left to right: intact tendon, sutured tendon 24 hours after surgery, 1 week after surgery and 8 weeks after surgery; scaffold-mediated healed tendon using piezoelectric and non-piezoelectric scaffolds after 8 weeks post-surgery. It is observed that the tissue organization in highly increased when using scaffolds and there is no presence of ectopic bone

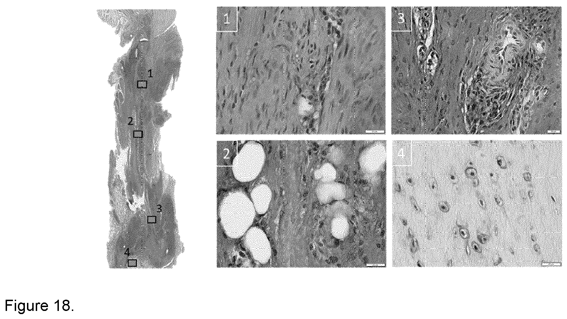

Figure 18 illustrates Alcian blue stained sample 2 weeks post tendon transection showing disruption of organized tissue when repair is mediated using sutures. 1) Upper cut end showing cells undergoing phenotype changes 2) Lipid deposition in the mid part of the injury 3) Vessel formation for cartilage formation at the lower cut end 4) Chondrocytic cells surrounded by their lacunae producing specific matrix deposition.

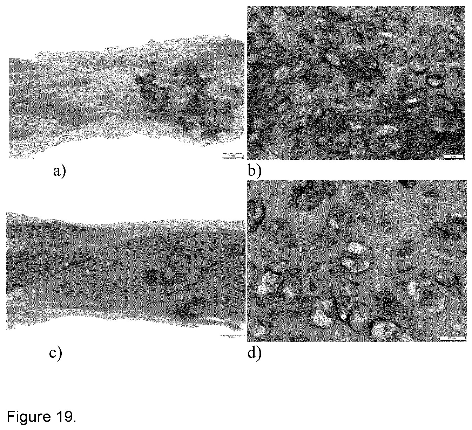

Figure 19 illustrates Alcian blue staining image from injury samples after a) 4 weeks and c) 8 weeks post injury. b) Typical discrete cartilage nodule formation appearance on the mid-part of the granulation tissue after 8 week post-injury. d) Image showing hypertrophic chondrocytes

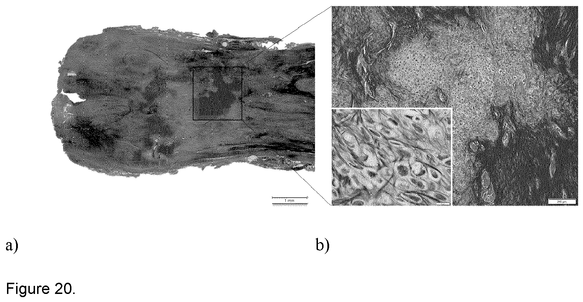

Figure 20 illustrates a) O-safranin stained tendon sample showing endochondral ossification after 8 weeks post implantation in injury group (static). b) Hypertrophic Chondrocytes are completely encapsulated by their lacunae and separated by ECM characteristic of endochondral ossification. Bone marrow cavities start appearing.

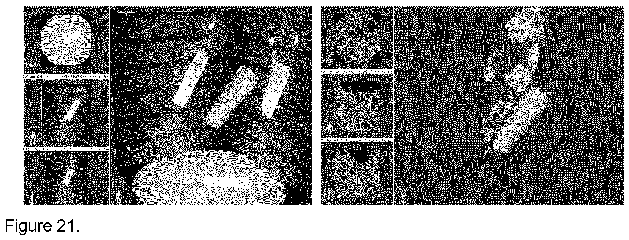

Figure 21 illustrates Miro CT scans of tissues repaired using a) non-piezoelectric scaffolds or b) piezoelectric scaffolds after 8 weeks post tendon transection (PTI). As expected it is possible to observe for both scans, mineralized tissue in the distal part of the tendon corresponding to the insertion of the tendon with the bone. However, for piezoelectric scaffolds, significant amount of mineralized tissue is detected surrounding the implant.

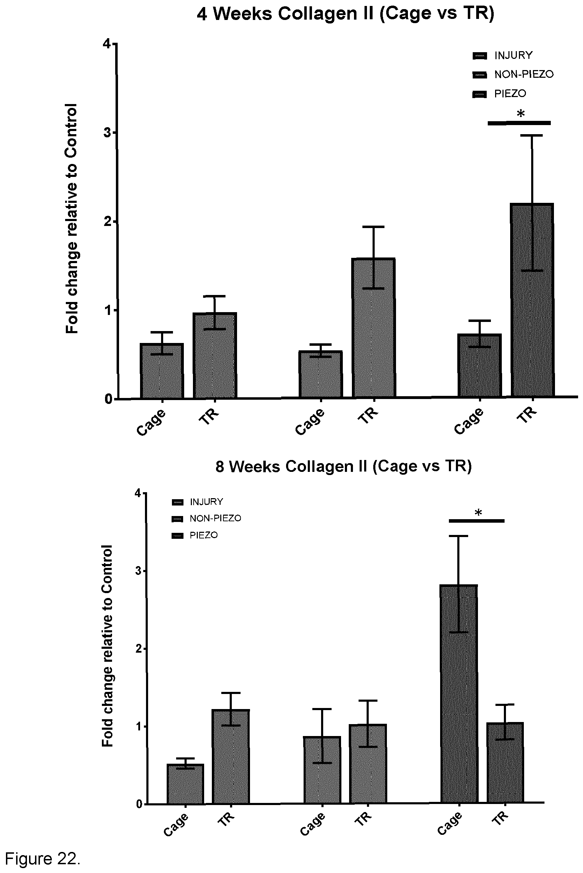

Figure 22 illustrates Collagen II expression comparison between animals non-running and running animals. (Mean $\pm$ SEM)It is observed that piezoelectric scaffolds reduce the ectopic formation under dynamic conditions.

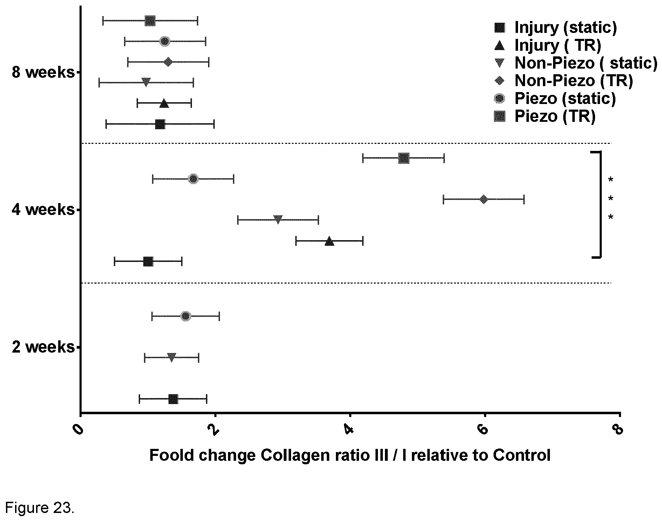

Figure 23 illustrates Fold change values of collagen III to I ratio relative to contralateral control at weeks 2, 4 and 8 after injury for all the experimental groups. The expression of collagen I and III was normalized first by the expression of $\beta$-actin and then by the contralateral control. (*** $p < 0.001$)

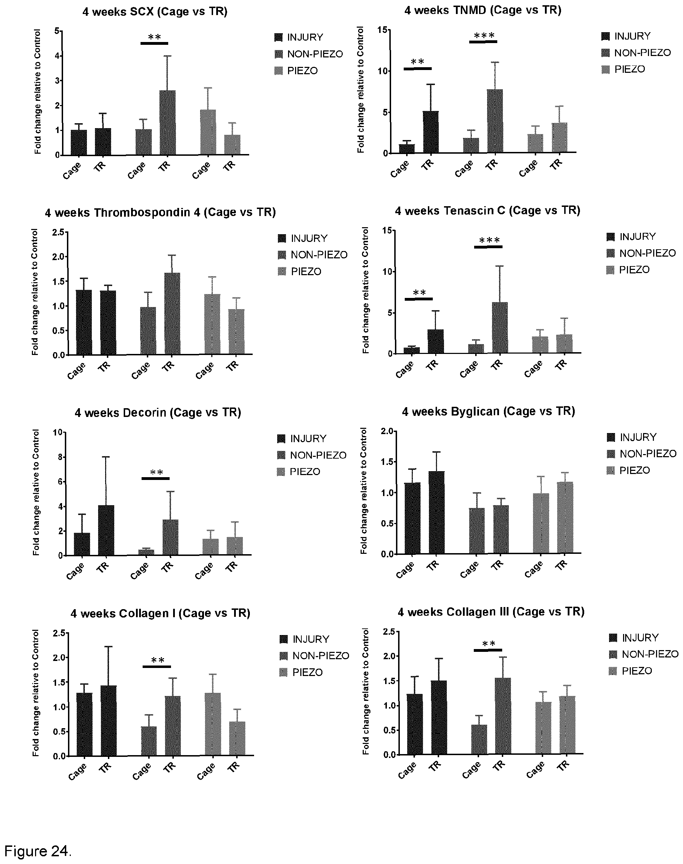

Figure 24 illustrates expression of tendon specific protein expression at 4 weeks after injury. Data represents mean $\pm$ SEM (n=4). Analysis performed using one-way ANOVA, *p<0.05. **p<0.01,***p<0.0001.

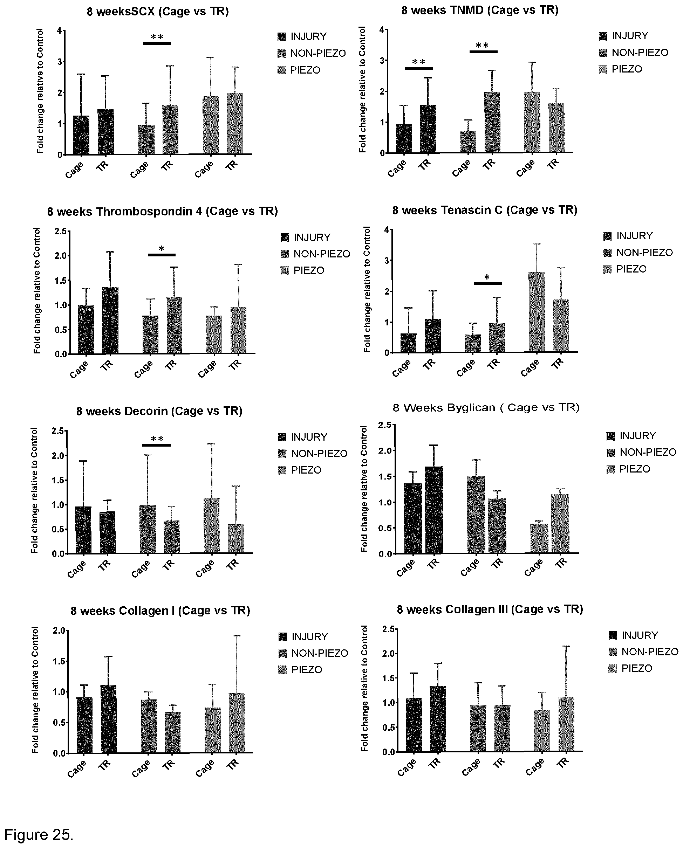

Figure 25 Expression of tendon specific protein expression at 8 weeks after injury. Data represents mean $\pm$ SEM (n=4). Analysis performed using one-way ANOVA, *p<0.05. **p<0.01, ***p<0.0001.

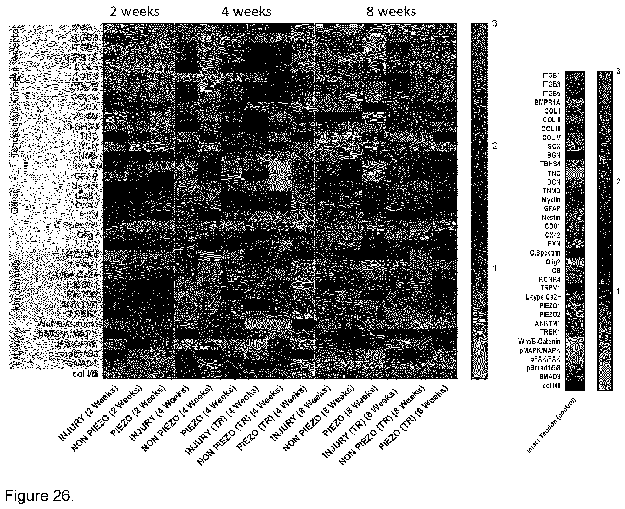

Figure 26 Effect of stimulation (mechanical and electromechanical) on the cellular receptors, function, ECM production and molecular mechanisms related to tenogenesis. Heat maps of proteins analyzed across samples collected from injury, non-piezo and piezo groups at weeks 2, 4 and 8 (static and TR) relative to control. The 35 proteins are represented in rows and classified in different groups according to their function. Data represents mean value of fold change relative first to $\beta$-actin and then to control sample (n=4). Intact tendon sample is represented by fold change mean value relative to $\beta$-actin of all contralateral samples collected at weeks 2, 4 and 8 for static and TR groups (n=21).

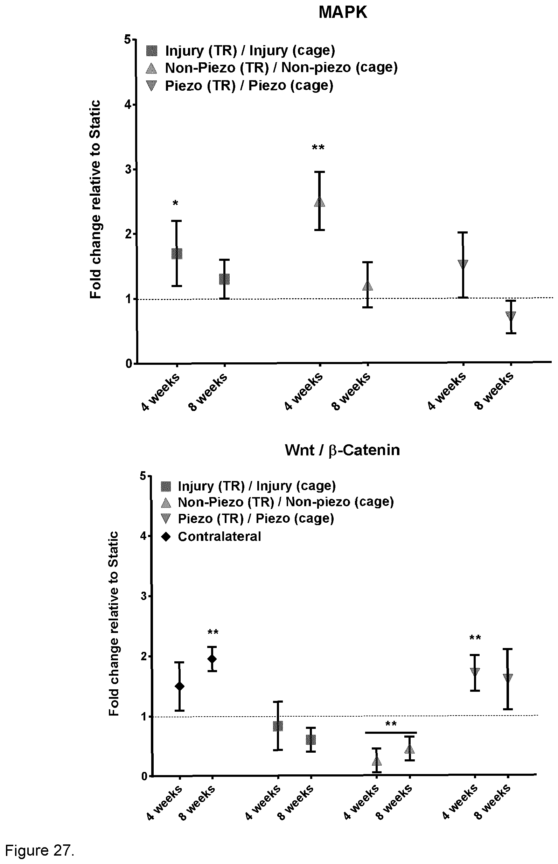

Figure 27 Graph showing the fold change in MAPK and Wnt/$\beta$-catenin expression of TR samples relative to cage samples for each group at weeks 4 and 8. Significance is shown between samples and activation level (mean=1).

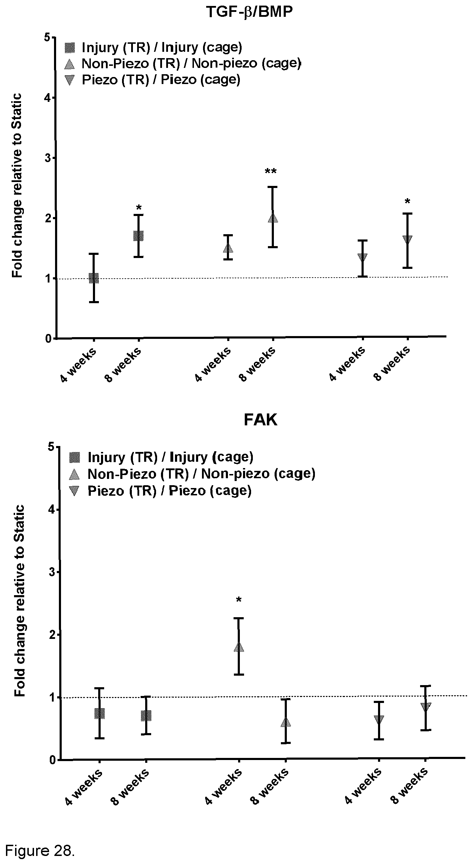

Figure 28 Graph showing the fold change in TGF-$\beta$/BMP and FAK expression of TR samples relative to cage samples for each group at weeks 4 and 8. Significance is shown between samples and activation level (mean=1).

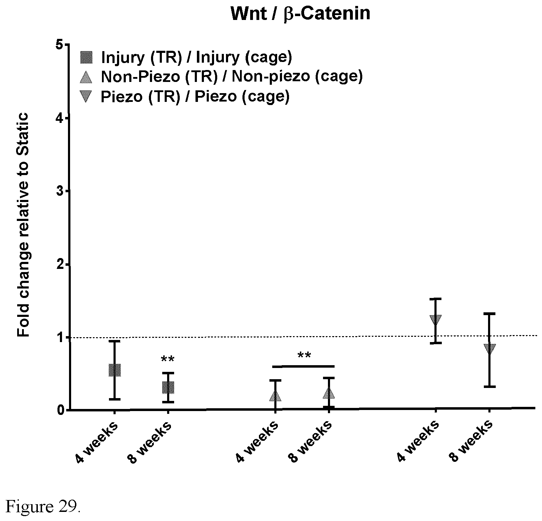

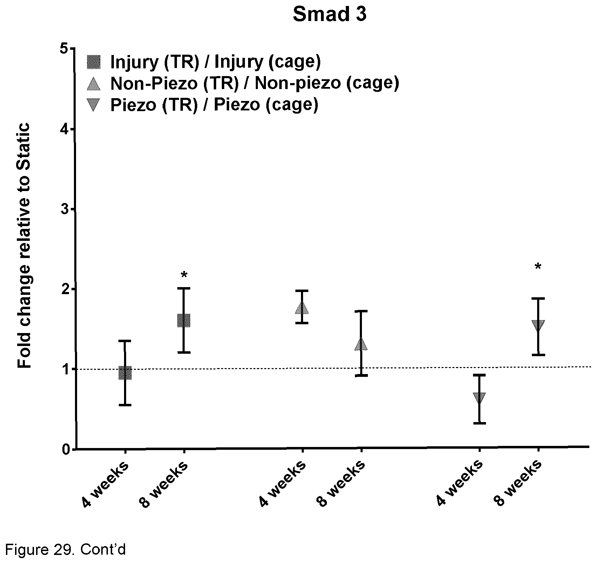

Figure 29 Graph showing the fold change in Smad3 and Wnt expression of TR samples relative to cage samples for each group at weeks 4 and 8. Significance is shown between samples and activation level (mean=1).

Detailed Description of the invention

A 3D tissue model of tendon repair that mimics the electrical and morphological properties of tendon tissue was developed through the alignment of electrospun fibres PVDF-TrFE (PVDF-TrFE chemical structure $-(CH_2-CF_2)_n$ lies in between polyethene (PE) $-(CH_2-CH_2)_n-$ and PTFE $-(CF_2-CF_2)_n-$ structures). The piezoelectric output was subsequently quantified under physiologically relevant dynamic mechanical tension to examine the effects of electromechanical modulation on tendon regeneration. It is known that the amplitude of electrical potentials generated in stressed tendon is dependent upon the rate and magnitude of tensile strain, while polarity is determined by the direction of loading. In many existing therapies, especially orthopaedic centered, electrical stimulation by direct and alternating electric currents using invasive or semi-invasive methods, pulsing electromagnetic fields and capacitive coupled electric fields, have all been observed to directly increase regeneration. Incorporation of a piezoelectric component, has been used by the inventors with an aim of incorporating dynamic electrical cues in response to applied mechanical loading can be used.

Example 1

1.1. Electrospinning

Typically, in an electrospinning process, a polymeric solution is held in a syringe connected to a high voltage power supply through a metallic capillary. When increasing voltage is applied, the surface tension of the polymer is overcome by the electrostatic forces of the following electric field and fibre is ejected from the capillary tip. At this moment the initially rounded shaped drop of polymer in solution elongates and forms a cone shape know as Taylor cone. When the electrical field reaches a level significant enough to break the surface tension forces, a fibre emerges from the tip of the cone and the electrically charged jet is then attracted to a collector. During its flight, the solvent from the polymeric solution is evaporated, and the fibre narrows until is deposited on the collector surface, which is generally grounded or partially charged with an opposite electrical charge to attract the fibres preferentially. Also, the collector cab rotates to organise the fibres in an aligned fashion during collection. Two types of parameters control the final morphological and chemical properties of the collected fibres, namely solution and working parameters. Solution parameters are those exclusively related to the polymer including molecular weight, concentration, solvent, conductivity and viscosity. Moreover, working parameters are those related to the electrospinning operation including flow rate, the distance of the electrode nozzle to the collector, collector rotational speed, applied voltage and nozzle diameter.

Electrospinning process

P(VDF-TrFE) and PTFE were made into fine fibres by varying the voltage, mandrel rotation speed and the distance between tip and collector with a constant flow rate. Each polymeric solution was placed in an Aladdin programmable syringe pump (AL-1000) (World Precision Instruments) and attached to a 27-gauge stainless steel needle. Aluminium foil was cut in a 10cm x 27/28cm portion and taped around the cylindrical mandrel collector (Linari Engineering S.r.l).
P(VDF-TrFE) 75/25 weight % (Solvay Solexis) was dissolved in a 3:2 volume ratio of dimethylformamide (DMF)/acetone (Sigma-Aldrich) at a polymer solvent concentration of 15-35% w/w. Briefly, 2 ml of solution was placed into a 10 ml plastic syringe (Luer lock). Any excess air remaining in the syringe was removed, and a stopper was placed on the syringe to prevent the sample from drying out. The syringe was placed 6 cm away from the collector, the flow rate was set constant at 1 ml/hour, and the collector speed was set at 3700 rpm. Finally, the voltage was switched on and increased up to 24-26kV. The collector was also charged with -6kV. The experiment ran for 2 hours, and lower humidity yielded more favourable results (40-50%).
PTFE was electrospun with a 2 step process. First, 240 milligrams of poly(ethylene oxide) (PEO), average $M_v$ □0300,000 (Sigma-Aldrich), was weighed out and mixed with 10 ml of PTFE, 60 wt % dispersion in $H_2O$ (Sigma-Aldrich). The syringe was clamped into a polymer blender and mixed gently for 2 hours. After blending, the air from the syringe was removed using centrifugation tube at 1500 RPM for 2 minutes. The solution was removed from the centrifuge and filtered through a syringe filter with 25 mm diameter (Merck Millipore) into a clean syringe at least twice to ensure no precipitates were present. The syringe was placed 10-12cm away from the collector, and the flow rate was pre-set at 1.25ml/hour. The rotator was set at the maximum velocity of 2000 RPM since fibres at higher speed tend to break due to their poor mechanical integrity. The voltage was set to 11/12 kV. The experiment ran for 4-5 hours, and a higher relative humidity yielded more favourable results (50-60 %). In the second step, PTFE mats were collected and annealed at 185 °C for 5 minutes.
A 30 kV potential difference between collector and nozzle tip (200 um), a flow rate of 1 ml/hr and a collector - tip distance of 6 cm was identified as the working parameters for optimal electrospinning of PVDF-TrFE. Also, a collector disk with 8 mm width and 8 cm diameter was used to concentrate the electrical field while rotating at 3500 rpm (30 m/s linear speed) to draw and align the collected fibres. The advantage of using large linear speeds is 1) it promotes the formation of mesoscopic joints between adjacent fibres (as result of residual no evaporated solvent due to short flight times) to maximise the mechanical properties of the scaffold and 2) it mechanically draws the fibres increasing their mechanical and electrical performance. DMAc was used in combination with acetone (50:50 v/v) since the surface morphology of the fibres is slightly rough and due to the high boiling point of DMAc (Tb: 165°C) it helps for the formation of fibre interconnections. The optimal averaged fibre diameter of the scaffolds used was measured as 540 nm $\pm$ 120 nm.

Strips of 8 mm width, 25 cm length and thickness between 27 and 75 um depending on the spinning time were obtained. The diameter of the nanofibres produced was around 540 nm with a narrow dispersion (see Figure 2).

Total alignment was calculated using Fast Fourier Transform (ImageJ) of scanning electron images of fibres organised randomly or in an aligned fashion. The alignment was constant along the entirety of the scaffold, and the porosity of the scaffolds was measured to be around 75 %. In general, fibre collection speed resulted in highly aligned scaffolds with higher rigidity. Other processing parameters were also explored including conductivity of the polymeric solution, plasma treatment effect and distance between collector and syringe.

To assess the effect of conductivity salt (NaCl) was incorporated into the solution. The main effect observed was changes of the surface morphology that might alter the mechanical strength of the scaffold. It is well recognised that surface roughness can generate defects nucleation and affects the mechanical strength of the material. Therefore, smoother surfaces are always preferred when looking to maximise mechanical properties. Conversely, roughness can increase cell attachment since more area for cellular adhesion is available and cells can sense nanoscale topographies. Prolonged exposure times to ion bombardment during plasma treatment results in high levels of nanoscale roughness and oxidation (incorporation of hydroxyl groups into the surface chemistry) that result in a change from hydrophobic to a hydrophilic character.

The effect of distance between syringe and collector was evaluated. Conventional electrospinning using long distance (20 cm) between collector and nozzle was used. Also, a systematic comparison of the effect of the distance across a broad range of fibre diameters was studied. In order to control fibre diameter, a range of different polymer concentrations was used. The concentration ranged from 11% to 35% to obtain a library of fibres between 180 and 540 nm.

The thermal transition properties of P(VDF-TrFE) scaffolds were evaluated using differential scanning calorimetry (DSC). PVDF-TrFE scaffolds demonstrated two main transitions: an initial endothermic peak, associated to the Curie temperature and a second endothermic peak that is associated to the melting temperature. Little differences were observed in the thermal transitions of the studied samples, indicating that the concentration of the polymer solution has a negligible effect on the thermal transition of the resulting scaffolds. Accordingly, all the samples showed a $T_c$ and $T_m$ at ~117°C and ~141°C, respectively. The observed $\Delta H_c$ and the $\Delta H_m$ values were ~5 J g$^{-1}$ and ~17 J g$^{-1}$ in all the studied samples.

FTIR spectra of PVDF-TrFE scaffolds suggested $\beta$-phase is the predominant crystalline phase in the PVDF-TrFE scaffolds. A clear trend was not observed between the concentration of the polymer solution and the $\beta$-phase content. Scaffolds with 390 nm (1.4), 490 nm (1.6) and 540 nm (1.7) fibre diameter had the highest $\beta$-phase content (73%; 78% and 72% respectively). Differently, scaffolds with the lowest fibre diameter (180 nm) or spun using long distance (between collector and needle) showed at the lowest $\beta$-phase content (59% and 58% respectively). Long distance scaffolds showed inferior mechanical performance compared to short distance electrospun scaffolds

When comparing the mechanical properties of samples with varying fibre diameter using short distance electrospinning, a three-fold increase in the elasticity modulus was obtained for scaffolds with fibre diameters ranging from 180 nm to 540 nm. Crystallinity did not appear to account for this increase in mechanical properties. Samples with 180 nm fibre diameter showed 44% crystallinity whereas scaffolds with 490 nm showed 53 % crystallinity. Similarly, the overall crystallinity for scaffolds with random fibres with a fibre diameter of 590 nm was 40%. It was considered that mechanical drawing during fibre collection at high rotational speeds resulted in enhancement of crystallinity level, in particular, the polar $\beta$-phase. An increase in mechanical properties of highly aligned scaffolds with a fibre diameter of 490 nm is considered to be a result of a high level of chain extension and orientation along the fibre axis as a result of fibres being drawn by the centripetal forces. Mechanical properties, to generate higher stresses under fixed strains (resulting in higher electromechanical/piezoelectric signals), were considered to depend on processing parameters including the distance between needle and syringe, rotational speed and polymer solution concentration.

Two post-processing processes, thermal annealing (cold crystallisation) and strain-hardening (cold-drawing) were also considered for controlling further the mechanical properties, crystallinity and $\beta$-phase content. In general, thermal annealing (over Tc temperature 132°C for 2h) resulted in highly crystalline samples (>70% as shown in chapter 2). The increase in crystallinity after annealing originates from increased $\gamma$-phase (15% on annealed samples compared to 1% on as-spun samples). However, under SEM visualisation, it was noticed that thermal annealing resulted in the loss of fibre organisation and introduced defects on the structure (in the form of cracks) due to thermal expansion coefficient changes between crystalline and amorphous phases. It was obtained that annealing at 90°C for 1 hour during fibronectin functionalization process was a good compromise. Higher annealing temperatures (i.e.110 to 135) had a negative impact on essential characteristics of the samples (fibre organisation and surface morphology, increased brittleness) for the indented tissue engineering (decreased porosity as fibre start forming more mesoscale and macroscale joints between the fibres and the total volume of scaffold was reduced by 30%).

Based on the initial properties of the scaffolds (45 % maximum elongation), drawing was performed at 12% and 20% to induce stress-hardening through plastic deformation of the scaffolds. The diameter of fibres was reduced from 540 nm to 490 nm after cold drawing at 20% strain and from 540 to 516 nm after cold drawing at 12 % strain. The elastic modulus was measured after 12% cold drawing and corresponded to 334 MPa/gr/cm$^2$. However, an increase in the scaffold mechanical strength (30 MPa/g/cm$^2$) and elasticity was at the cost of toughness that was reduced from 24 J/gr to 15 J/gr.

Scaffolds were functionalized to covalently attach fibronectin their surface

*Functionalizing electrospun material*

Post electrospinning, due to sample chemistry the sample material are functionalized (adding specific integrin binding moieties) before use in cell culture as a biomaterial scaffold. The samples were carefully removed from the aluminium foil and placed on a square sample holder. Three functional chemistries were used: Collagen (50$\mu$g/ml), Poly-L-lysine (PLL) (10$\mu$g/ml) and fibronectin (FN) (20$\mu$g/ml) to aid in the promotion of cell attachment.

Since the biological activity of proteins on the surface depends upon whether specific active peptide sequences are accessible to the cells, the accessibility of the cell-binding domain was ensured by the covalent binding of fibronectin to modified surfaces using Poly-Acrylic Acid (PAAc) as a spacer

Example 2. Scaffold Fabrication

PVDF-TrFE (piezo) or PTFE)non-piezo) were fabricated into tubular scaffold composed of aligned fibers via electrospinning. Briefly, PVDF-TrGE or PTFE solution was delivered at a flow of 1.0 ml/hr using an 18G Luer-lock needle. A +20 kV potential difference between needle and collector at a distance of 6-10 cm was used and fibers were collected onto a negatively charged mandrel (-6kV) rotating 4000 rpm.

Aligned nanofibres P(VDF-TrFe) electrospun onto a rotating collector produced elastic piezoelectric scaffolds. DSC, tensile and SEM analysis showed that short distance electrospinning yielded high alignment at the micro and molecular level, thereby avoiding the need for post-treatment processes (i.e. electrical poling and mechanical drawing) to enable a high piezoelectric response (i.e. increase in beta phase formation).

Uniaxial stretching was used to activate a piezoelectric response of PDVF-TrFE scaffolds at constant frequency/strain rate of 0.5 Hz and strain amplitude of 4%. Human tenocytes cultured on mechanically loaded piezoelectric scaffolds exhibited a positive functional response after 5 and 10 days of piezostimulation relative to mechanically loaded non-piezoelectric scaffolds.

Although aligned fibers scaffolds promoted cell elongation (relative to non-aligned scaffolds), nuclear shape was not significantly changed either in static or dynamic conditions.

Mechanical forces were determined to play a determinant role in modulating the function and phenotype of tendon cells. After application of mechanical forces, intense tenogenic and osteogenic differentiation processes were initiated through activation of mechanosensitive receptors including action channel TRPA1 and focal adhesion genes.

Piezoelectricity reinforces cell adhesion by differentially activating key adhesion receptors such as integrins and focal adhesion kinase (FAK). Importantly, it was shown that piezolectrical control of cell function was result of interplay between TGF/BMP signaling and direct osteospecific pathways such as Wnt signaling. Piezoelectricity mediated changes in the morphology and cell adhesion also modulated indirect intracellular signaling including MAPK/ERK and ROCK/RhoA via activation of piezosensitive channels receptors including Piezo 1&2.

Tendon derived cells responded to piezoelectricity and a piezoelectric scaffold may be fabricated capable of promoting tendon tissue repair.

Tendon is a highly specialized tissue with extreme mechanosensitive characteristics, capable of coordinating its structure and composition when is mechanically challenged. However, an often overlooked characteristic of tendon is its piezoelectric behaviour under dynamic physiological conditions. The electrical response to the changing mechanical environment is believed to play a fundamental role in controlling cell response to orchestrate the downstream signaling cascade for starting the tissue remodeling process. The inventors used piezoelectric scaffolds to replicate the natural piezoelectric characteristic of tendon and assess their regenerative capacity in a tendinopathy animal model. In addition, different mechanotransductive pathways were studied to unravel the effect of piezoelectricity during tendon repair.

Tendon response to overuse/intense treadmill running regimes during healing results in cartilage formation or ossification with higher expression levels of collagen II, sox-9, aggrecan, osterix or runx-2 and characteristic histological changes including intense collagen staining, lipid deposition, loss of fiber organization, calcification, vascularity and increased number of dedifferentiated cells (phenotypic drift). Conversely, following moderate exercise, the rate of synthesis of collagen I/III and levels of growth factors (i.e. TGF-$\beta$) is increased. The effect of moderate exercise conditioning on wound healing has been also tested using a rat moderate treadmill running model. It was observed that rats preconditioned with MTR before injury were able to heal faster tendon wounds than non-running rats. In this model, collagen I/III synthesis and organization was promoted as well as tendon cell function (increase in tenomodulin and collagen I) compared to "static" model (non-running rats) that was characterized by osteochondral formations (increase in Runx2 and collagen II) in the wound. In addition, it has been shown that MTR can increase the expression of stem cell markers (nestin and nanog), tenocyte gene expression (TNMD and SCX) and decreases lipid deposition, proteoglycan accumulation, calcification and expression of non-tenocyte markers. By comparing between animals undergoing moderate treadmill running and non-running, changes in the ECM can be expected and using protein arrays the molecular mechanism can be uncovered.

Therefore, a system to stimulate functional tendon repair is moderate exercise in form of moderate treadmill running. In this study, chosen moderate treadmill running protocol was treadmill running 5 days a week for 30 to 60 min at a speed between 10 to 13 m/min. The results in our model are consistent with the literature and demonstrated a strong positive effect of treadmill running on the functional recovery of animals with injured tendons. Animals repaired using sutures and undergoing treadmill running showed a faster recovery compared to animals in cages as shown in the recovery of MTP joint displacements. However, the most significant benefits were found when scaffolds and treadmill were used synergistically to repair tendon. When using non-piezoelectric scaffolds and treadmill running, the repair was enhanced in as little as 4 weeks and the effect was more evident after 8 weeks. Similarly, piezoelectric scaffolds and treadmill running showed an incremental functional recovery after 4 and 8 weeks. Treadmill running was studied on the tissue reorganization, composition and ectopic bone formation after injury. The results showed that collagen II was initially increased in animals undergoing treadmill running but after a period of 8 weeks the treadmill running groups showed lower levels of expression of collagen II and reduced levels of ectopic bone formation. Interestingly, the most significant modulation was obtained when using piezoelectric scaffolds and treadmill running. Similarly, synthesis of collagen I and III were also upregulated as consequence of the continuous mechanical stimulation and repair using scaffolds. It was noticed that the collagen III to collagen I was very high after 4 weeks indicative of the proliferative stage of wound healing. However, the levels were normalized after 8 weeks characteristic of the early remodeling phase. To quantify the differences obtained a modified tendon healing scoring was used. The results demonstrated that the group with more mature repaired tendon was the non-piezoelectric (TR) followed by the piezoelectric (TR) group. The score obtained for all the (TR) groups was significantly higher than that of cage groups. Similarly, tendon related markers Tenomodulin, Scleraxis, Thrombospondin 4 and Tenascin-C were significantly upregulated in the non-piezo (TR) group at week 4 and 8 and this trend was also followed by the rest of TR groups but not significantly. Finally, measurements of cartilage and bone formation using histomorphometry suggested a reduction of ectopic bone formation in animals undergoing treadmill running and was further confirmed by micro CT scanning. These results are consistent with similar studies conducted in rats and mices that reached the conclusion that increasing exercise activity delays bone formation in a tendon injury model. Despite these studies did not investigate systematically the mechanism underlying, they proved that bone formation is related to the activation of the Wnt/β-catenin signaling pathway. In this work, the aim was to investigate the pathways involved in tendon repair using piezoelectric and mechanical stimulation after tendon injury.

It was shown that the peak of functional recovery at week 4 in the non-piezo (TR) group (mechanical stimulation) was accompanied by the activation of the FGF-MAPK/ERK and TGF-β/BMP signaling pathway. Conversely, the pathways associated to piezoelectric stimulation (piezoelectric (TR) group) were FAK and Wnt/β-catenin signaling pathways. Furthermore, cage groups also showed a significant activation of the Wnt/β-catenin signalling pathway relative to control group. According to a growing body of research, Wnt/β-catenin signaling plays a pivotal role in tissue regeneration and development. A number of studies have suggested a direct 'link between mechanical stress and activation of the Wnt pathway that enhances bone healing and regeneration. Even though, it is commonly believed that activation of bone formation related signaling pathways might be not desired during tendon healing, it becomes central for tendon to bone insertion. It was shown that the higher levels of activation of this pathway were obtained when using piezoelectric scaffolds in static conditions and decreased when mechanically stimulated. These results suggest that electrical charges might promote the activation of this pathway and bone formation. Hence, a transient activation of this pathway might enhance tendon to bone healing; however, a prolonged activation might become detrimental for the functional tendon repair.

Scaffold-mediated tendon repair resulted in smaller size and reduced ectopic bone formation and enhanced matured tendon tissue. Furthermore, enhanced tendon tissue regeneration was obtained when non piezoelectric mechanically stimulated. In contrast, when using piezoelectric scaffolds bone formation was promoted but decreased when mechanically stimulated. In conclusion, it becomes clear that tendon tissue remodeling can be modulated by mechanical and electrical stresses.

The results showed that functional tendon repair follows the activation of TGF-β and FGF/MAPK-ERK pathways together with TRP-like ANKTM1 and Piezo2 ion channels and integrins beta 1 and 5. Conversely, activation of Wnt/β-catenin resulted in ectopic bone formation and deactivation of Piezo2 and TRPV1 ion channels and integrin beta 1.

*In vivo implantation*

All the animal procedures and treatments used in this study were approved by the ethics committee at the National University of Ireland, Galway. In addition, animal care and management followed the Standard Operating Procedures of the Animal Facility of the National University of Ireland, Galway. Animals were allowed to acclimatize for at least seven days prior to any surgical procedures. Subsequently, animals were acclimated to the treadmill running for one week and their behaviour was analysed.

A total of 105 Female Lewis rates aged 6-8 (220g) weeks were used in this study. The animals were anaesthetized by isofluorane inhalation (5% induction reducing to 1-2% for maintenance during procedures). The right leg was shaved and swabbed with iodine to minimise the risk of bacterial contamination. An incision was created through the skin (~1cm) from the myotendinous junction distally to the osteotendinous junction. The incision provided ample exposure of the Achilles tendon. The fascia surrounding the Achilles was transected longitudinally and carefully to avoid creating injury and the Achilles tendon was exposed. Prior to implantation and tendon transection, two looped sutures are inserted at the top (muscle) and bottom (bone). After the total tendon length was measured a 3 mm defect in proximal/distal extension (at 3 mm from the calcaneus) was created using a positioning device and an 11 surgical blade, resulting in a 6 mm gap after tissue retraction. The construct was then sutured (4-0 Ethicon) to the both ends of the tendon to bridge the gap using a modified Kessler technique and the skin was sutured. After a period of 2, 4 and 8 weeks the animals were euthanized and tendon tissue as well as contralateral tendons were harvested.

Animals were then left to heal for at least 2 weeks and after were gradually exposed to a treadmill running (see Table 2) based on published studies where non adverse effects on the tendon morphology were detected respect to healthy rat Achilles tendon without training. It is reported that beyond these values tendon would be considered overused and risk of tear would be increased. Briefly, the treadmill running was increased from once a week for 5 min to 30-45 min 5 days a week.

Table 1. Running protocol used for the running group of rats (treadmill)

Table 1. Running protocol used for the running group of rats (treadmill)

| | | Duration (min) | Speed (m/min) |
|---|---|---|---|
| | Day 14 | 5 | 8-9 |
| | Day 15 | 10 | 9-10 |
| Week 2 | Day 16 | 15 | 9-10 |
| | Day 17 | 20 | 9-10 |
| | Day 18 | 30 | 9-10 |
| | Day 19 | 45 | 9-11 |
| | Day 20 | 30 | 9-11 |
| Week 3 | Day 21 | 45 | 9-12 |
| | Day 22 | 30 | 10-14 |
| | Day 23 | 45 | 10-14 |
| Week 4 | | 30 | 10-14 |
| Week 5 | | 45 | 10-14 |
| Week 6 | | 30 | 10-14 |
| Week 7 | | 45 | 10-14 |
| Week 8 | | 30 | 10-14 |
| Week 9 | | 45 | 10-14 |
| Week 10-16 | | 30 | 10-14 |

*Histology*

For histologic analyses, repaired tendon tissues and contralateral tendons of all groups (n=7) were dissected from the proximal myotendinous junction to the distal osteotendinous junction and processed. The samples were fixed in 10% neutral buffered formalin (24 hours), dehydrated using an alcohol gradient, cleared, and embedded in paraffin blocks, as reported previously. Histological sections (6 um thick) were prepared using a microtome. In order to distinguish between scar tissue or new tendon formation, polarization microscopy and picrosirius red staining were used. In addition, for descriptive histology 6 μm thick sections were stained using Hematoxylin & Eosin stain, Masson-Goldner's stain, Alcian Blue, O-safranin stain, Red picrosirius satin or Herovici's polychrome stain according to the manufacturer's guidelines. Areas of chondrification within the defect region at 4 or 8 weeks after surgery were measured using Safranin O staining and Fiji (ImageJ v1.52i). For each tissue 3 different frontal-longitudinal sections were analysed (1 section of the middle part, 1 section ventral of the middle part and 1 section dorsal of the middle part). For each section 5 consecutive images were captured spanning the entire length of the repair tissue, omitting the transition zones of original tendon stumps to repair tissue. Volume fraction (VV) of tendon cells was used to estimate the cell proliferation.

A 192-point grid was overlaid on 40X images of H&E stained tissue sections. The number of tendon cells intersecting points of the grid is counted (PP), along with the total number of points on the tissue (PT). The volume fraction of tendon cells (VV) is calculated using the formula below:

$$V_V = P_P / P_T$$

Histomorphometry and point-based scoring system.

In order to evaluate the progress of tendon repair and the effect of treadmill running, histomorphometric measurements on stained samples from three animals (5 slides in total per animal) were employed. A point-based scoring system was used and the following parameters were scored based on the percentage of: 1) increase of number of cells compared to intact tendon 2) Increase of calcification compared to intact tendon 3) Increase on the vascularization and innervation compared to intact tendon 4) Increase of fat deposits compared to intact tendon 4) Increase of fiber orientation compared to intact tendon 5) Increase of cell morphology compared to intact tendon.

*Micro-CT scan*

At 8 weeks after Achilles tenotomy, the right hind legs of each group were collected at sacrifice for micro-computed tomography analyses (μCT 100, Scanco Medical, Bruttisellen, Zurich, Switzerland). Samples were scanned using the following settings: 60 kV, 150 μA with a mean 20 μm slice thickness. The reconstructed scaffold and Achilles tendons were selected for quantification of calcifications volume.

*Protein array*

Tissue samples were immediately frozen at -80 °C until needed. Samples were then pulverized in a cell crasher for five minutes at least three times until the tissue was completely powder. Sample powder were thawed on ice and incubated in a lysis buffer containing a cocktail of protease and phosphatase inhibitors for five minutes at least three times and centrifuged at 15,000 g for 15 minutes. The protein fraction of the centrifuged sample was then extracted, aliquoted and stored at -80 °C until further use.

The protein expression in the newly formed tendon tissues was examined using custom made array technology. Protein arrays were fabricated following the protocol described in chapter 3 and 4 and used to analyze the effect of electromechanical stimulation in ECM production.

*Functional recovery*

In this study, an animal treadmill running (Exer 3/6, Columbus Instruments) track integrated with a video-based system was used to obtain spatiotemporal parameters of gait. The animal gait was analysed through clear plastic Lexan at the sides of the system consisting of a cage (50.8 cm x 50.8 cm x 33 cm) with gates placed at each end of the walkway. A light was directed from the anterior direction of the walkway to prevent the rats from stop running. A digital camera (8 M pixels and 120 frames per seconds) was positioned 30 cm in front of the walkway to capture the sagittal view of the rat from the walkway. The data was analyzed and processed by Kinovea software (v0.8.27). For all groups, the system was calibrated using the same scale bar located in the image. The system model the leg motion using pairs of circle markers.

The ankle, knee, hip and MTP joint angles were measured using the automated black ink marker feature recognition on the hip, knee, ankle, and 4rd metatarsal head at the four gait stages: initial contact, mid-stance, pre-swing, and mid-swing. The spatial and temporal gait parameters analyzed were; step length, angle and cycle time. The walking speed was calculated by dividing the step length by the cycle time. Each angle joint curve was normalized by cycle time before further analysis. To determine the change on gait angle, the maximum difference in amplitude (Ampl.) between initial contact and end of swing was measured during an entire step and was calculated according to following formula:

$$Max.(º) - Min.(º) = Ampl.(º)$$

Statistical Analysis

Minitab (v.17 Minitab Software) was used for statistical analysis. Analysis of variance (ANOVA) and Tukey's post-hoc test were used to determine statistical significance between groups. All graphical data was presented as mean $\pm$ standard deviation of mean. p values of < 0.05 were considered statistically significant.

Developing a preclinical load-bearing injury model was necessary to investigate the effect of piezoelectric scaffolds during tendon repair. Therefore, a full thickness transection of the tendon of 3 mm was performed in the rat Achilles tendon.

Under normal conditions, in intact tendons, cells are well elongated and imbibed in a rich and dense ECM composed of predominantly collagen type I crosslinked by GAGs. However, after an injury occurs or age-related degeneration, resident and invading cells tend to differentiate and adopt different phenotype resulting in a tendinopathy.

Phenotypic drift of cells

In intact tendons bundles of cells elongate longitudinally along collagen fibers showing a very specific phenotype characteristic of tenocytes. Conversely, after 2 weeks post injury fibroblastic cells present in the granulation tissue yet conserving the longitudinal alignment are deployed of the characteristic phenotype of tenocytic cells.

Macroscopically, injured tendon samples (in average) were significantly thicker in diameter than intact tendon. Table 2 shows the morphometric values of the repaired tendons and intact tendons.

Table 2. Diameter of tendons after injury in animals undergoing treadmill running (dynamic) or just left in the cage (static)

|  | 2 weeks | 4 weeks | 8 weeks |
| --- | --- | --- | --- |
| Intact tendon | 1.6 $\pm$ 0.2 mm | 1.6 $\pm$ 0.2 mm | 1.7 $\pm$ 0.2 mm |
| Injury (static) | 5.4 $\pm$ 0.5 mm | 4.9 $\pm$ 0.4 mm | 4.6 $\pm$ 0.4 mm |
| Injury (TR) | 5.4 $\pm$ 0.4 mm | 4.3 $\pm$ 0.5 mm | 3.8 $\pm$ 0.8 mm |
| Non-piezo (static) | 5.1 $\pm$ 0.4 mm | 4.1 $\pm$ 0.6 mm | 3.4 $\pm$ 0.7 mm |
| Non-piezo (TR) | 5.1 $\pm$ 0.2 mm | 4.0 $\pm$ 0.3 mm | 3.3 $\pm$ 0.5 mm |
| Piezo (static) | 5.5 $\pm$ 0.6 mm | 4.1 $\pm$ 0.6 mm | 4.1 $\pm$ 0.9 mm |
| Piezo (TR) | 5.2 $\pm$ 0.7 mm | 4.1 $\pm$ 0.4 mm | 3.9 $\pm$ 0.9 mm |

Furthermore, the functional recovery of the injured tendons was measured using gait analyses of animals walking in a treadmill at a low speed of 9 m/min. The gait of animals was analyzed using a custom-made system and the data is presented in the Table . Gait analysis were conducted once pre-surgery and at 1, 2, 4 and 8 weeks after Achilles tenotomy. The gait analyses tracked the joint side angles of the knee, MTP, hip and ankle.

Right after injury (1 week), animals were not showing any recovery and they adopted an antalgic gait by decreasing the MTP joint angle, increasing the hip join angle and increasing the time in stance by 20-30%. Conversely, after 2 weeks post injury all animals were able to utilize and load the tendon.

Table 3. Functional recovery analyses over a period of 8 weeks. It is observed that the use of a scaffold promotes a faster recovery of the leg motion (N=7).

| | | Injury (static) | | | | Non-piezo (static) | | | | Piezo (static) | | | |
|---|---|---|---|---|---|---|---|---|---|---|---|---|---|
| | Pre-surgery (º) | 1 week (º) | 2 weeks (º) | 4 weeks (º) | 8 weeks (º) | 1 week (º) | 2 weeks (º) | 4 weeks (º) | 8 weeks (º) | 1 weeks (º) | 2 weeks (º) | 4 weeks (º) | 8 weeks (º) |
| MTP | 100±2 | 32±4 | 51±6 | 52±5 | 51±7 | 42±4 | 54±3 | 56±5 | 55±9 | 40±4 | 53±8 | 55±5 | 52±8 |
| Knee | 80±3 | 49±5 | 54±8 | 53±4 | 63±7 | 55±5 | 55±10 | 55±4 | 55±2 | 45±5 | 54±9 | 56±10 | 54±6 |
| Hip | 18±2 | 16±2 | 15±5 | 12±4 | 9±4 | 14±2 | 13±5 | 10±1 | 12±5 | 17±3 | 14±7 | 10±3 | 8±6 |
| Ankle | 70±2 | 41±5 | 56±3 | 57±11 | 58±7 | 48±6 | 57±3 | 53±7 | 51±5 | 43±4 | 68±6 | 55±9 | 55±5 |

Table 4. Functional recovery analyses over a period of 8 weeks on animals undergoing treadmill running. It is observed a significant differences between the animals undergoing treadmill running and left in the cage (N=7).

| | Pre-surgery (º) | Injury (TR) | | Non-piezo (TR) | | Piezo (TR) | |
| --- | --- | --- | --- | --- | --- | --- | --- |
| | | 4 weeks (º) | 8 weeks (º) | 4 weeks (º) | 8 weeks (º) | 4 weeks (º) | 8 weeks (º) |
| MTP | 100±2 | 48±3 | 71±8 | 58±5 | 92±8 | 48±5 | 62±8 |
| Knee | 80±3 | 58±7 | 67±7 | 62±3 | 72±3 | 60±7 | 64±6 |
| Hip | 18±2 | 15±5 | 15±5 | 14±2 | 15±3 | 8±2 | 12±8 |
| Ankle | 70±2 | 55±3 | 61±3 | 54±4 | 67±3 | 57±3 | 67±7 |

It was observed that the patterns/profiles of the gait did not differ from the experimental groups to the control (pre-surgery) samples (see Figure). However, a considerable difference in the joint angle amplitudes was measured. First, it was noted that the joint angle amplitudes were at their lowest values one week after surgery compared to control group (pre-surgery) except for the hip joint angle amplitude that was at its highest. In addition, all the experimental amplitudes for ankle, MTP and knee joint angles were significantly lower in the repaired groups than the control group at weeks 1, 2, 4 and 8 ($p < 0.05$).

Notably, gait analyses results suggested that treadmill running had a consistent significant positive effect, in particular on the group non-piezo (TR) at week 4 ($p<0.05$) for the ankle and at week 8 for ankle, knee and MTP joints ($p<0.01$, see Figure). It is also important to note that after 8 weeks, the recovery of MTP angle was complete ($p<0.01$) demonstrating an enhanced functional recovery and tendon repair. Similar trend was also observed for the piezoelectric scaffold group undergoing treadmill running significantly just for knee joint angle at week 8 ($p<0.05$).

In summary, scaffold mediated repaired showed a full functional recovery as fast as 4 weeks for non-piezoelectric scaffolds group undergoing treadmill running and partially complete functional recovery for piezoelectric scaffolds group undergoing treadmill running at week 8.

Enhanced tendon healing and tissue organization is observed on scaffold-mediated repair compared to suture-aided repair.

After injury, the tendon stumps retract generating a gap that is filled with granulation tissue. The fibrous repaired tissue showed loose collagen fiber organization and an overall heterogeneous texture after 2, 4 and 8 weeks, with increased cellularity and ingrowth of vessels, nerves, fat deposition and calcifications.

Briefly, after 1 week post injury, the newly formed tissue is composed of mainly disorganized granulation tissue comprised of collagen III. After 2 weeks post injury, the tissue composition was highly heterogeneous with increased cellularoty and incrowth of nerves and vessels. After 4 weeks, condensation of cells showing chondrogenic phenotype in both injury and scaffold treated samples was observed. Inevitably, after 4 weeks post injury the process of chondrogenesis was initiated due to the mechanical compression and hypoxic conditions experienced by resident and infiltrating cells in especially in the bone and muscle insertion areas. Indeed, the major complication observed after removing the mid-part of the tendon was endochondral ossification after 8 weeks post injury. The Figure shows the dedifferentiation of tendon cells into chondrocytic cells forming cartilage nodules at the end of the stumps and in the mid-part of the granulation tissue. As newly formed chondrocytes progress in their maturation, the cartilage nodules are eventually replaced by mineralized bone.

In order to evaluate the progress of tendon repair and the effect treadmill running, histomorphometric measurements on stained samples from three animals (5 slides in total per animal) were employed. A point-based scoring system was used and the following parameters were scored based on the percentage of: 1) increase of number of cells compared to intact tendon 2) Increase of calcification compared to intact tendon 3) Increase on the vascularization and innervation compared to intact tendon 4) Increase of fat deposits compared to intact tendon 4) Increase of fiber orientation compared to intact tendon 5) Increase of cell morphology compared to intact tendon.

A clear sign of enhanced tendon maturation is the increase on birefrigerence of thick and organized collagen I fibers. Using polarized light microscopy on picrosirius red stained samples, it was confirmed that healing mediated by scaffolds (piezo and non-piezo) had a significant increase on the maturation and organization of collagen fibers as indicated by fiber organization score (>3) compared to suture (1.5). Furthermore, the decrease of cartilage related glycosaminoglycan's (GAGs) detected using safranin-O staining intensity measurements suggested also a significant ($p<0.05$) superior maturation level on the non-piezo (TR) groups compared to the other study groups.

Table 5. Scoring system for evaluation of the histological structures after repair for 8 weeks. Values are expressed in Mean $\pm$ SEM of scored points. 0-25 % (4 points); 26-50 (3 points); 51-75% (2 points) and 76-100% (1 point).

| Parameters | | | | | | | |
|---|---|---|---|---|---|---|---|
| Group | Cellular activity | Calcified area | Vessels and nerves | Fat deposits | Fibre organization | Cell morphology | Total |
| Control | 4 ± 0 | 4 ± 0 | 3.5 ±0.25 | 3.5 ± 0.5 | 3.75 ± 0.25 | 4 ± 0 | **23** |
| Injury | 1 ± 0.25 | 2 ± 0.5 | 3.25 ± 0.5 | 2.5 ± 0.5 | 1.5 ± 0.5 | 2.5 ± 0.5 | **12.75** |
| Injury (TR) | 1 ± 0.5 | 2.5 ± 0.25 | 2.5 ± 0.5 | 2.5 ± 0.5 | 2.5 ± 0.5 | 2.5 ± 0.5 | **13.5** |
| NP | 1 ± 0.75 | 2.25 ± 0.5 | 2.5 ± 0.5 | 2.5 ± 0.5 | 3.0 ± 0.5 | 2.5 ± 0.5 | **13.5** |
| NP (TR) | 2 ± 0.5 | 2.5 ± 0.5 | 3.25 ± 0.5 | 2.5 ± 0.5 | 3.25 ± 0.75 | 3 ± 0.5 | **16.5** |
| P | 1 ± 0.75 | 1 ± 0.5 | 2.5 ± 0.5 | 2.25 ± 0.5 | 2.0 ± 0.75 | 2.5 ± 0.5 | **12.5** |
| P (TR) | 1.5 ± 1 | 2.25 ± 0.5 | 2.5 ± 0.25 | 2.5 ± 0.5 | 3.25 ± 1.25 | 2.5 ± 0.75 | **15.0** |

From all the groups evaluated, the samples with better tissue organization and higher score were the non-piezoelectric (TR) and piezoelectric (TR) experimental groups. According to the results obtained from the scoring system, the non-piezoelectric (TR) group showed a significant (p<0.01) higher score (16.5) and the piezoelectric (TR) showed a significant high score (p<0.01) compared to the rest of the groups (see Table), indicating a superior tissue structure and healing process.

*Non-piezoelectric scaffolds reduce endochondral ossification but piezoelectric scaffolds enhance ectopic bone formation in static conditions (cage).*

Histologically, endochondral ossification involves four stages: inflammation, chondrogenesis, osteogenesis and maturation. First, during inflammation, immune cells invade the injury site and infiltrate. After, endothelial cells from the surrounding epitenon undergo endothelial to mesenchymal transition and acquire stem cell-like phenotype. During inflammatory process post-injury, cells from the epitenon and muscle start proliferating and invade the injury site along with immune cells that clear the space from broken-down collagen fibers disrupting the highly organized structure. A recent study demonstrated that Scx[+] tendon-derived and interstitial muscle derived Mkx1[+] progenitor cells mediate chondrogenesis by differentiation into chondrocytes. After 2 weeks post-injury empty spaces were still present between the large collagen type I and cells start showing phenotypic drift while the gap created was filled with granulation tissue.

Following inflammation (after 2 weeks post-injury), tendon structure reorganization is initiated and areas of high cell density were observed specially close to the stumps endings. Cartilage formation first appeared at the distal region of stump ending. After 4 weeks post injury, a large number of cartilage nodules were formed in the region of granulation tissue close to the distal stump ending. Within this region, the chondrocytes matured into hypertrophic chondrocytes and mineralization of the tissue started. Several small cartilage nodules were present from 4 weeks both at the tendon stumps and throughout the granulation tissue. Progressively, cartilage nodules served as template for new bone formation via endochondral ossification. Finally, ectopic bone formation results from hypertrophy of chondrocytes and blood vessel infiltration. In addition, under histological examination of the newly formed bone, it was possible to observe bone marrow cells within the marrow cavity.

To confirm ectopic bone tissue after 8 weeks post injury, micro CT scanning was used on all the different samples. It was demonstrated the presence of mineralized tissue around the implants. In addition, the highest volume of calcified tissue was obtained for repaired tissues mediated by piezoelectric scaffolds under static conditions (cage).

Piezoelectric stimulation modulates collagen synthesis and tendon-related proteins expression

After treadmill running the early cartilage formation was considerably modulated as shown by the expression of collagen II. Following an initial increase of collagen II expression consistently across all treadmill groups at week 4, the trend was stopped after 8 weeks and even reversed when using piezoelectric scaffolds.

Collagens are the main bulk constituent of tendon tissue. In particular, Collagen type I is associated to tissues under tensile loads and is the main responsible for tendon mechanical strength and durability. Increase of synthesis of collagen I over collagen III is a good indicator of tendon tissue maturation. During embryonic tendon tissue development, thin and immature disorganized collagen III fibers mature into thick collagen I fibers with strong birefringence and highly aligned organization. Conversely, Collagen II represents almost 80% of the total collagen content in cartilage and is specialized in supporting compressive forces. In tendon, collagen II just appears in small amount in the regions near the bone whereas fibrils of collagen III are always associate to collagen I. It was observed that the synthesis of collagens, in particular collagen III, was significant at week 4.

During wound healing, early granulation tissue is produced by fibroblasts to act as a temporary scaffold for cell attachment and proliferation. Generally, granulation tissue is mainly comprised of collagen III and low levels of collagen I. As the healing continues, the ratio of collagen I / collagen III becomes higher but generally never reaches its original values (around 1.4). It was observed a significant increase on the collagen type III to collagen I ratio after 4 weeks for all TR groups and the ratio decreased significantly after 8 weeks ($p < 0.001$).

During maturation, the tissue gets more resilient and mechanically strong via cross linking of the organized collagenous network by incorporation of GAGs / proteoglycan including Biglycan and Decorin. Therefore, analysis of Decorin and Byglican level expression were analyzed. It was observed that Decorin (DCN) was significantly modulated along increased levels of SCX and TNMD at weeks 4 and 8 for the non-piezoelectric scaffolds group (TR) ($p < 0.05$). Despite similar trend was observed for piezoelectric scaffolds (TR) group the effect was not as significant at either weeks 4 and 8 ($p > 0.05$). Tesnascin-C is an important glycoprotein that is used as a marker for tendon tissue embryonic organization. Furthermore, tenascin-C is normally associated after mechanical injury including overload of tendons. Tenascin-C was significantly increased at 4 and 8 weeks in the non-piezo (TR) group ($p < 0.05$) indicating that tenascin-C may act also as a mechanosensitive protein that modulates tissue organization.

The role of piezoelectricity in mechanotransductive pathways.

Ectopic bone formation is inhibited by deactivation of Wnt/β-catenin and activation of FAK signaling pathways.

Over the last century, it has been recognized the clear link between biomechanics and function of tissues. However, even nowadays the exact mechanism of how cells can sense and respond biochemically to mechanical forces remains elusive. There is an increasing body of research showing that cells dispose of a number of molecular machineries for sensing distinct type of mechanical stimuli with extreme high spatiotemporal resolution. The biological response can range from cytoskeletal reorganization, adhesion to ECM rearrangements or complete cell reprogramming. At the tissue level, the transient mechanical perturbations results in coordination of the tissue structure and composition of the interstitial matrix (remodeling) to reach an homeostatic balance between mechanical loading and tissue mechanics. It is believed that tissue remodeling is orchestrated at the cellular level by mechanical stresses. In this study, two type of different protein families have been studied as "active sensors" of mechanical stresses to initiate biological responses. The first group is adhesion related proteins including integrins (integrin β1, β3 and β5), BMP receptors signaling proteins (Paxilin). The second group studied was transmembrane ion channel proteins (including KCNK4, TRPV1, TREK1, ANKTM, Piezo1&2 and $Ca^{2+}$ L-type) sensitive to mechanical and electrical stimuli.

The most significant functional response of healed tendon tissues was obtained for the groups using mechanically stimulated non-piezoelectric and piezoelectric scaffolds. Additionally, the use of mechanical loading stimulation by treadmill running during repair resulted in significant tissue structure and composition adaption at week 4 through upregulation of collagens synthesis and tendon-related molecules such as Tenomodulin (TNMD), Tenascin-C (TNC) and Scleraxis (SCX). It has been shown in a number of studies that synthesis of collagen is regulated by various cytokines and signaling molecules (TGF-β, Smad, Wnt, MAPK and FAK). The activation of these signal transduction pathways were studied by looking at the tyrosine phosphorylation state of the pathway related protein.

The results obtained showed an increased activation of the Piezo2 and ANKTM1 ion channels at week 2 and 4 for non-piezo (TR) group relative to cage. The activation of these ion channels was positively correlated with the activation of FGF-ERK/MAPK and FAK pathway ($p > 0.01$). Other significantly modulated molecules were collagen I and V, Scleraxis, Tenascin-C, Tenomodulin, CD81, BMPR1A, chondroitin sulphate (CS), Paxillin and integrins β1 and 5 that were upregulated at week 4.

Similarly, the molecular pathways associated to ectopic formation of bone after injury in static groups was also studied. It was observed a strong correlation between the static groups with higher levels of Wnt/β-catenin at week 4 ($p < 0.01$). In addition, it was observed that piezoelectrical scaffolds at static and dynamic (TR) conditions were associated with transient activation of Wnt/β-catenin at week 8 ($p < 0.01$) and 2 ($p < 0.01$) compared to the non-piezo (static) group that showed prolonged activation (see Figure) at weeks 2, 4 and 8 ($p < 0.01$). In addition, high ectopic bone formation was obtained when using piezoelectric scaffolds (cage) and was associated to dowregulation of tendon related proteins including SCX, COL I/III, SMAD3, TNC and DCN and upregulation of collagen II. Interestingly, this changes were also associated to deactivation of integrin beta 1, TRPV1 and Piezo2

Finally, activation of Smad-dependent pathway (TGF-β/BMP) was observed in (TR) groups at weeks 4 and 8 compared to their corresponding static group ($p < 0.05$). As shown in Figure, upregulation of Smad3 and activation of TGF/BMP signaling pathway are naturally involved in the homeostasis of native tendon. During normal (healthy tendon) structural adaptation, collagen I to collagen III ratio was 1.4 and the main upregulated proteins were integrins β3 and 5, BMPR1A, Collagen I and V, Thrombospondin 4, Decorin and Tenomodulin.

In summary, mechanical loading of tendon tissues in vivo resulted in upregulation of mechanotransductive pathways and collagen synthesis, and ectopic bone formation was correlated with prolonged activation of Wnt/β-catenin and scaffolds under static conditions. Interestingly, when piezoelectric scaffolds were dynamically stimulated, ectopic bone formation was reduced and associated with transient modulation of Wnt/β-catenin and smad3 (see Figure 29).

**Claims**

1. A tendon or ligament repair implant comprising a biocompatible scaffold, wherein the scaffold is formed from electro-spun PTFE wherein
the electro-spun PTFE comprises a plurality of aligned nanoscale PTFE fibres.

2. An implant according to claim 1, wherein the alignment of the fibres is at least 90%.

3. An implant according to any one of the preceding claims, wherein the scaffold has a percentage elongation of at least 90 % and a tensile strength of between 1.35 to 8 MPa.

4. An implant according to any one of the preceding claims, wherein an outer surface of the scaffold comprises hydrophilic groups, optionally wherein the hydrophilic groups are acrylic acid.

5. An implant according to any one of the preceding claims, wherein the scaffold further comprises a polyacrylic acid outer layer.

6. A method of preparing a tendon or ligament repair implant, the method comprising

    a) mixing a dispersion of PTFE particles in water with a fibre forming agent,
    b) electrospinning the mixture of a) onto a collector; and
    c) sintering the electrospun material to form a biocompatible scaffold.

7. A method according to claim 6, wherein the fibre forming agent is poly(ethylene oxide) (PEO) or PVA.

8. A method of claim 7 wherein the fibre forming agent has an average molecular weight of between 1 to 1,000,000 kDa.

9. A method according to any one of claims 6 or 7, wherein electrospinning the material comprises discharging the mixture from a needle of a syringe at a flow rate of no more than 1.25 mL/hr, optionally 1.0 mL/hr.

10. A method according to any one of claims 6 to 9, wherein the step of sintering the electrospun material comprises:

    a) heating at 10 °C/min to 350°C and holding for at least 5 minutes; and
    b) heating at 10 °C/min to 385°C and holding for at least 5 minutes.

11. A method according to any of claims 6 to 10 wherein there is provide a step of drying the electrospun material prior to sintering.

12. A method according to step 11 wherein the step of drying the electrospun material is drying for 2 to 24 hours at 70°C.

13. A method according to any one of claims 6 to 12, comprising a functionalising step wherein functionalising the outer surface of the scaffold comprises:

    treating the surface with oxygen plasma;
    immersing the plasma treated samples in methanol to wet the surface; and
    heating the samples under reflux in an aqueous solution of acrylic acid.

14. A method of drawing the PTFE scaffold comprising the steps:

    - extruding the electrospun PTFE having a predetermined thickness and length, a proximal end and a distal end, the proximal end having a temperature;
    - drawing the distal end to a predetermined length while maintaining the temperature of the scaffold below the glass transition temperature of PTFE during the drawing, the wall thickness of the proximal and distal ends form a waist

P(VDF-TrFE) copolymer

PTFE polymer

a)

PVDF    TrFE

b)

PVDF-TrFE

Figure 1.

500 rpm

3500 rpm

500 rpm
3500 rpm

FFT Intensity (a.u)

0   30   60   90   120   150   180
Polar Angle (°)

Figure 2.

a)

B

Figure 3.

a)

b)

Figure 4.

Figure 5.

a) PVDF-TrFE Surface

b) Hydroperoxidase groups

— OOH
— OOH
— OOH
— OOH

c ) Radical Polymerization

AAC

d) Carboxylic acid groups

— COOH
— COOH
— COOH
— COOH

e) EDC

Fibronectin

f) Covalently bonded Fibronectin

Figure 6.

a)                      b)                    c)

Figure 7.

Figure 8.

Figure 9.

a)          b)          c)          e)          f)          g)

Figure 10.

Figure 11. a)                                                     b

Figure 12.

a)

b)

Figure 12 c.

Figure 13.

EP 3 851 129 A1

a)

b)                              c)                              d)

Figure 14.

Figure 15.

Figure 15 Cont'd

Figure 15 Cont'd

Figure 16.

a)

b)

| Injury (sutures) | Injury (Scaffold) | No Injury (Intact tendon) |

Figure 17.

Figure 18.

Figure 19.

a)                                              b)

Figure 20.

Figure 21.

Figure 22.

Figure 23.

Figure 24.

Figure 25.

Figure 26.

## MAPK

## Wnt / β-Catenin

Figure 27.

## TGF-β/BMP

## FAK

Figure 28.

Figure 29.

Figure 29. Cont'd

Europäisches
Patentamt

European
Patent Office

Office européen
des brevets

# EUROPEAN SEARCH REPORT

**Application Number**

EP 20 15 2766

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | C W Bolton: "The GORE-TEX(TM) expanded polytetrafluoroethylene prosthetic ligament", Clinical orthopaedics and related research, 1 January 1985 (1985-01-01), pages 2012-2014, XP055710323, Retrieved from the Internet: URL:https://journals.lww.com/clinorthop/Abstract/1985/06000/The_GORE_TEXTM_Expanded_Polytetrafluoroethylene.27.aspx [retrieved on 2020-06-30] * page 177, column 2, lines 5-21 * ----- | 1-14 | INV. A61L27/16 A61L27/56 |
| A | US 2019/271098 A1 (JOHNSON JED K [US] ET AL) 5 September 2019 (2019-09-05) * claims 1, 4 * * paragraph [0029] * ----- | 1-14 | |
| A | WO 2016/138221 A1 (MERIT MEDICAL SYSTEMS INC [US]) 1 September 2016 (2016-09-01) * paragraphs [0039] - [0040] * ----- | 1-14 | TECHNICAL FIELDS SEARCHED (IPC) A61L |

The present search report has been drawn up for all claims

| Place of search | Date of completion of the search | Examiner |
|---|---|---|
| The Hague | 2 July 2020 | Guazzelli, Giuditta |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 3 851 129 A1**

## ANNEX TO THE EUROPEAN SEARCH REPORT
## ON EUROPEAN PATENT APPLICATION NO.

EP 20 15 2766

This annex lists the patent family members relating to the patent documents cited in the above-mentioned European search report.
The members are as contained in the European Patent Office EDP file on
The European Patent Office is in no way liable for these particulars which are merely given for the purpose of information.

02-07-2020

| Patent document cited in search report | | Publication date | Patent family member(s) | | Publication date |
|---|---|---|---|---|---|
| US 2019271098 | A1 | 05-09-2019 | NONE | | |
| WO 2016138221 | A1 | 01-09-2016 | CA | 2975391 A1 | 01-09-2016 |
| | | | EP | 3261589 A1 | 03-01-2018 |
| | | | JP | 2018506365 A | 08-03-2018 |
| | | | KR | 20170122733 A | 06-11-2017 |
| | | | US | 2016250048 A1 | 01-09-2016 |
| | | | US | 2019008665 A1 | 10-01-2019 |
| | | | WO | 2016138221 A1 | 01-09-2016 |

For more details about this annex : see Official Journal of the European Patent Office, No. 12/82